# EUROPEAN PATENT APPLICATION

(11) **EP 4 722 240 A1**
(43) Date of publication of application: **08.04.2026**
(21) Application number: 24306641.2
(22) Date of filing: 07.10.2024
(51) Int. Cl.: C07K 16/16, A61P 35/00, C07K 16/28, C07K 16/30, C07K 16/40

(54) **COMBINATION OF ANTIBODIES, OR ANTIGEN-BINDING FRAGMENTS THEREOF, FOR THE TREATMENT OF CANCERS AND COMPOSITIONS THEREOF**

(71) Applicant: Xenothera, 44200 Nantes (FR)
(72) Inventor: BASSISSI, Firas, 44120 VERTOU (FR); CIRON, Carine, 44330 LE PALAIS (FR); DAUPHOUY, Ophélie, 44000 NANTES (FR)
(74) Representative: Ipsilon

(57) **Abstract**

The present invention relates to a combination of antibodies, or antigen-binding fragments thereof, for use in the treatment of a cancer in a human subject in need thereof, the combination comprising at least a first antibody, or an antigen-binding fragment thereof, specifically binding to MVP; and (i) at least an antibody (a), or an antigen-binding fragment thereof, specifically binding to at least an antigen selected from the group consisting of BCAP31, PEPD, PAXX, ASPH and mutated KRAS subtype KRAS^{G12D}; and/or (ii) at least an antibody (b), or an antigen-binding fragment thereof, specifically binding to at least an antigen selected from the group consisting of SERPINB6, EGFR, CEACAM6 and RNPEP. The present invention further relates to a pharmaceutical composition for use in the treatment of a cancer in a human subject in need thereof, comprising this combination of antibodies, or antigen-binding fragments thereof, and a pharmaceutically acceptable carrier.

## Description

### Field of the invention

The present invention relates to the field of treatment of cancers. In particular, the present invention relates to the field of using polyclonal antibody compositions or combinations of antibodies directed against specific antigens and their implementation in the treatment of cancers.

### Description of Related Art

Cancer remains a leading cause of death worldwide, with nearly 20 million new cases and an estimated 9.7 million cancer-related deaths reported in 2022. Globally, about 1 in 6 deaths is due to cancer. Inaccessible or insufficiently active treatments are common in this field, highlighting the well-recognized need to develop new and improved therapies for treating various cancers. Among the many genetic alterations implicated in cancer, mutations in the KRAS gene are significant, contributing to tumorigenesis and posing unique challenges in treatment. KRAS encodes a small GTPase that regulates critical signaling pathways for cell growth and survival. Mutations at codons G12, G13, and Q61 lead to constitutive activation of KRAS, resulting in uncontrolled cell division, particularly in aggressive cancers such as pancreatic cancer, non-small cell lung cancer (NSCLC), and colorectal cancer.

Despite considerable advancements in cancer treatment, including targeted therapies, many patients still face resistance to existing therapies, underscoring the urgent need for innovative approaches. While recent developments in covalent inhibitors targeting specific KRAS mutations have shown promise, the majority of cancer patients remain in need of effective treatment options. Additionally, the genomic heterogeneity of tumors contributes to variability in treatment responses and the rapid development of resistance. This highlights the critical importance of advancing therapeutic strategies that can address a broad spectrum of cancers and their associated resistance mechanisms.

Immunotherapies represent a real hope in the treatment of a great number of cancers, in particular in the treatment of cancers not currently efficiently cured by conventional therapies. This treatment mainly consists in the administration of monoclonal antibodies directed against tumor cells or directed against activators or inhibitors of a checkpoint of the immune response against cancers. In addition to these immunomodulating monoclonal antibodies (mAb), antibody drug conjugate (ADC), chimeric antigen receptor T cells (CAR-T) and more recently bispecific antibodies are being actively explored and successfully introduced as innovative weapon in cancer treatment arsenal. However, for many cancers, there is still a lack of effective treatments, especially treatments that can result in long-term cancer-free survival and lower metastatic and relapse risks.

Antibodies directed against tumors (passive immunotherapy) can act through two complementary ways:
- by cytotoxicity complement dependent (CDC), dependent from killer cells (ADCC) or dependent from phagocytes (ADCP) and apoptosis;
- by induction of an adaptive immune response. It has indeed been demonstrated that opsonization of a target and the local production of complement's molecules (C3a, C5a) (see Strainic et al., Immunity 28(3):425-35 (2008)) activates the T lymphocyte co-stimulation and increases its survival. Thus, the passive administration of antitumor antibodies can, as a first step, generate the discharge of factors from the complement that, in a second step, ease the T cells' response.

Monoclonal antibodies have revolutionized the treatment of various cancers, providing targeted therapy that enhances patient outcomes. However, a significant challenge remains in the form of treatment resistance, which affects a substantial number of patients. As such, 40% to 85% of the patients are resistant to treatments based on the use of monoclonal antibodies. This resistance to monoclonal antibody therapies can arise through several mechanisms, including the activation of alternative signaling pathways, mutations in the target antigen, and changes in tumor microenvironment that promote immune evasion. As a result, patients may experience disease progression despite initial responses to treatment, leading to a decrease in overall survival rates. This underscores the urgent need for innovative therapeutic approaches that can effectively address resistance mechanisms and improve treatment efficacy for those patients who are currently not responding to available monoclonal antibody therapies. Accordingly, a treatment based on the use of combinations of antibodies targeting different antigens on the tumoral cells might allow minimizing the escaping mechanisms.

However, despite their potential efficacy as observed by Richet more than 100 years ago, combinations of antibodies, such as polyclonal antibodies of animal origin, have rarely been used in the treatment of tumors due in particular to high toxic risks in patients. This toxicity is mainly linked to the expression of Neu5GC and alpha-1,3-galactose carbohydrates on animal immunoglobulins which in human elicit potent anti-Neu5GC and anti-alpha-1,3-galactose immune responses associated with allergy, serum sickness disease, and formation of immune complexes. In order to mimic the polyclonal humoral immune response for cancer treatment, combinations of monoclonal have been considered. However, their clinical efficacy as monotherapy does not predicate their safety and clinical efficacy in combination (Berlin et al., Investigational New Drugs 40:586-595 (2022)). The difficulty of such combination mainly lies in the association of antibodies with different PKs (which makes it more difficult to control toxicity), the stability of the product according to the isotypes and the manufacturing (see for example Larbouret et al., Cancers (Basel) 13(18):4620 (2021)).

This invention seeks to address these critical gaps in treatment by leveraging the therapeutic potential of polyclonal antibodies.

Unlike monoclonal antibodies, which target a single epitope, polyclonal antibodies have the ability to recognize and bind to multiple epitopes on a tumor cell. This broad specificity enhances their therapeutic efficacy by enabling more robust and sustained targeting of cancer cells, which can be particularly advantageous in heterogeneous and mutationally diverse tumors. Polyclonal antibodies can also engage multiple immune pathways, improving their ability to recruit the immune system to attack cancer cells, thereby overcoming some of the limitations seen with single-target therapies. Polyclonal antibodies, by targeting multiple epitopes, provide a broader and potentially more effective therapeutic strategy against these heterogeneous and aggressive cancers.

There accordingly remains a need in the field for novel anti-cancer therapeutic agents, and in particular novel therapeutic agents efficient against a great variety of cancers.

There accordingly remains a need in the field for novel anti-cancer therapeutic agents, and in particular novel therapeutic agents efficient against a plurality of liver, pancreatic, colorectal and lung cancers.

There also remains a need in the art for the provision of an effective and improved therapy for treating cancers and endowed with reduced or no adverse effects.

There also remains a need in the art for the provision of novel anti-cancer therapeutic agents efficient against cancers carrying at least a KRAS mutation, and in particular against lung, pancreatic and/or colorectal cancers carrying at least one KRAS mutation, in particular KRAS^{G12A}, KRAS^{G12C}, KRAS^{G12D}, KRAS^{G12S}, KRAS^{G12V}, KRAS^{G13C}, KRAS^{G13D} and KRAS^{G12R} mutation(s), more particularly KRAS^{G12D}, KRAS^{G12C}, and KRAS^{G12V} mutation(s).

There also remains a need in the art for the provision of novel anti-cancer therapeutic agents efficient against liver, pancreatic, colorectal and lung cancers that are refractory to conventional treatments.

There also remains a need in the art for the provision of novel anti-cancer therapeutic agents able to activate complement and to activate apoptotic mechanisms to kill cancer cells.

There also remains a need in the art for the provision of novel anti-cancer therapeutic agents able to provide at least an antitumoral activity selected from the group consisting of complement dependent cytotoxicity (CDC), global cytotoxicity, apoptotic activity, impaired spheroid formation, decreased tumor size, tumor invasion and/or metastasis.

The invention aims to meet the above-mentioned needs.

### Summary of the invention

The present invention in particular relates to the following items:
**Item 1:** A combination of antibodies, or antigen-binding fragments thereof, for use in the treatment of a cancer in a human subject in need thereof, the combination comprising:
   (i) at least a first antibody, or an antigen-binding fragment thereof, specifically binding to MVP (Major vault protein); and
   (ii) at least an antibody (a), or an antigen-binding fragment thereof, specifically binding to at least an antigen selected from the group consisting of BCAP31 (B cell receptor-associated protein 31), PEPD (Peptidase D), PAXX (Paralog of x-ray repair cross-complementing protein 4 (XRCC4) and XRCC4-like factor (XLF)), ASPH (Aspartate β-hydroxylase) and mutated KRAS (Kirsten rat sarcoma viral oncogene homologue) subtype KRAS^{G12D}; and/or
   (iii) at least an antibody (b), or an antigen-binding fragment thereof, specifically binding to at least an antigen selected from the group consisting of SERPINB6 (Serine protease inhibitor B6), EGFR (Epidermal growth factor receptor), CEACAM6 (Carcinoembryonic antigen-related cell adhesion molecule 6) and RNPEP (Arginyl aminopeptidase (aminopeptidase B)).
**Item 2:** The combination for use according to item 1, comprising:
   (i) at least a first antibody, or an antigen-binding fragment thereof, specifically binding to MVP; and
   (ii) at least an antibody (a), or an antigen-binding fragment thereof, specifically binding to at least an antigen selected from the group consisting of BCAP31 (B cell receptor-associated protein 31), PEPD (Peptidase D), PAXX (Paralog of x-ray repair cross-complementing protein 4 (XRCC4) and XRCC4-like factor (XLF)), ASPH (Aspartate β-hydroxylase) and mutated KRAS (Kirsten rat sarcoma viral oncogene homologue) subtype KRAS^{G12D}; or
   (iii) at least an antibody (b), or an antigen-binding fragment thereof, specifically binding to at least an antigen selected from the group consisting of SERPINB6 (Serine protease inhibitor B6), EGFR (Epidermal growth factor receptor), CEACAM6 (Carcinoembryonic antigen-related cell adhesion molecule 6) and RNPEP (Arginyl aminopeptidase (aminopeptidase B)).
**Item 3:** The combination for use according to item 1 or 2, comprising:
   (i) at least a first antibody, or an antigen-binding fragment thereof, specifically binding to MVP; and
   (ii) a/ at least an antibody, or an antigen-binding fragment thereof, specifically binding to BCAP31, and
      b/ at least an antibody, or an antigen-binding fragment thereof, specifically binding to PEPD, and
      c/ at least an antibody, or an antigen-binding fragment thereof, specifically binding to PAXX, and
      d/ at least an antibody, or an antigen-binding fragment thereof, specifically binding to ASPH, and
      e/ at least an antibody, or an antigen-binding fragment thereof, specifically binding to mutated KRAS subtype KRAS^{G12D}; and/or
   (iii) a/ at least an antibody, or an antigen-binding fragment thereof, specifically binding to SERPINB6, and
      b/ at least an antibody, or an antigen-binding fragment thereof, specifically binding to EGFR, and
      c/ at least an antibody, or an antigen-binding fragment thereof, specifically binding to CEACAM6, and
      d/ at least an antibody, or an antigen-binding fragment thereof, specifically binding to RNPEP.
**Item 4:** The combination for use according to any one of items 1 to 3, wherein the antigen-binding fragments of the antibodies present in the combination are independently Fv, Fab, F(ab')2, Fab', dsFv, (dsFv)2, scFv, sc(Fv)2, or diabodies.
**Item 5:** The combination for use according to any one of items 1 to 4, wherein the cancer is selected from the group consisting of myeloma; melanoma; breast cancer, in particular triple negative breast cancer; prostate cancer; primary and metastatic colorectal cancer, in particular colon cancer or metastatic colon cancer; lung cancer, in particular non small cell lung cancer (NSCLC) or small cell lung cancer (SCLC); liver cancer, in particular hepatocarcinoma or cholangiocarcinoma; primary and metastatic pancreas cancer, in particular pancreatic adenocarcinoma; lymphoma, in particular B-cell or T-cell lymphoma, more particularly T-cell lymphoma; gastric cancer; head and neck cancer; ovarian cancer; sarcoma; acute and chronic leukemia; osteosarcoma; anal cancer; testicular cancer; uterus cancer; thyroid cancer; cancer of the central nervous system; gastrointestinal stromal cancer; epidermal carcinoma and oesophageal cancer.
**Item 6:** The combination for use according to any of items 1 to 5, wherein the cancer is selected from the group consisting of pancreas cancer, in particular pancreatic adenocarcinoma; liver cancer, in particular hepatocarcinoma or cholangiocarcinoma; colorectal cancer, in particular metastatic colon cancer; and lung cancer, in particular non small cell lung cancer (NSCLC).
**Item 7:** The combination for use according to any of items 1 to 6, wherein the cancer comprises cancer cells harboring at least one KRAS mutation.
**Item 8:** The combination for use according to item 7, wherein the at least one KRAS mutation is selected from the group consisting of KRAS^{G12A}, KRAS^{G12C}, KRAS^{G12D}, KRAS^{G12S}, KRAS^{G12V}, KRAS^{G13C} and KRAS^{G13D}; in particular is selected from the group consisting of KRAS^{G12D}, KRAS^{G12C} and KRAS^{G12V}; more particularly is KRAS^{G12D}.
**Item 9:** The combination for use according to any one of items 1 to 8, wherein at least one of the antibodies of the combination, and in particular all the antibodies of the combination, is/are devoid of at least one antigenic determinant selected from (i) N-glycolylneuraminic acid (Neu5Gc) and (ii) α-1,3-galactose, and in particular is/are devoid of the two antigenic determinants N-glycolylneuraminic acid (Neu5Gc) and α-1,3-galactose.
**Item 10:** The combination for use according to any one of items 1 to 9, wherein at least one of the antibodies of the combination, and in particular all the antibodies of the combination, is/are Immunoglobulin G antibodies.
**Item 11:** The combination for use according to any one of items 1 to 10, wherein the combination is a combination of monoclonal antibodies or a polyclonal antibody composition.
**Item 12:** The combination for use according to item 11, wherein the polyclonal antibody composition is obtainable by immunization of an animal with pancreatic tumor cell antigens comprising KRAS mutated antigens.
**Item 13:** The combination for use according to item 12, wherein the animal is a non-human mammal, in particular a non-human mammal selected from the group consisting of rodents, such as mice, rats, guinea pigs and hamsters; lagomorphs, such as rabbits; ferrets; felines, such as cats; canines, such as dogs; goats; sheep; bovines, such as cows; swines, such as pigs and hogs; camelids; horses; and non-human primates.
**Item 14:** The combination for use according to item 13, wherein the non-human mammal is selected from the group consisting of lagomorphs, in particular rabbits; and pigs, and in particular is a rabbit or a pig lacking at least one gene selected in a group comprising (i) a gene encoding a functional cytidine-5'-monophosphate N-acetyl neuraminic acid hydrolase (CMAH) and (ii) a gene encoding a functional α-(1,3)-galactosyltransferase, and more particularly lacking both (i) a gene encoding a functional cytidine-5'-monophosphate N-acetyl neuraminic acid hydrolase (CMAH) and (ii) a gene encoding a functional α-(1,3)-galactosyltransferase.
**Item 15:** The combination for use according to any one of items 1 to 4 and 9 to 14, wherein the combination is included in a pharmaceutical composition further comprising a pharmaceutically acceptable carrier.
**Item 16:** The combination for use according to item 15, wherein the pharmaceutical composition further comprises at least one anticancer drug different from the antibodies of the combination of antibodies as defined in any one of items 1 to 4 and 9 to 14.
**Item 17:** The combination for use according to item 16, wherein the additional anticancer drug is selected from the group consisting of monoclonal antibodies, in particular selected from the group consisting of anti-CD19, anti-CD20, anti-CD30, anti-CD137, anti-CTLA4, anti-TIM-3, anti-B7-H3, anti-CD123, anti-CD134, anti-CD154, anti-LAG-3, anti-CD227, anti-BTNA3, anti-CD39, anti-CD73, anti-CD115, anti-CD47, anti-SIRP alpha, anti-SIRP gamma, anti-CD28, anti-NCR, anti-NKp46, anti-NKp30, anti-NKp44, anti-NKG2D, anti-PD1, anti-PDL1, mogamulizumab, obinutuzumab, polatuzumab vedotin, Yttrium Y 90-ibritumomab tiuxetan, mosunetuzumab, cetuximab, atezolizumab/bevacizumab, anti-VEGF antibodies, anti-DNAM-1 monoclonal antibodies and mixtures thereof.
**Item 18:** The combination for use according to any one of items 15 to 17, wherein the pharmaceutical composition further comprises at least one chemotherapy treatment, in particular a chemotherapy treatment, selected from the group consisting of a chemotherapy regimen consisting of Cyclophosphamide, Hydroxydaunorubicin, Oncovin and Prednisone (CHOP); a chemotherapy regimen consisting of Cyclophosphamide, Hydroxydaunorubicin, Oncovin, Etoposide and Prednisone (CHOEP); HDAC inhibitors; ibrutinib; acalabrutinib; zanubrutinib; copanlisib; venetoclax; folfirinox; cisplatin; doxorubicin; irinotecan; liposomal irinotecan; oxaliplatin; Nap paclitaxel; paclitaxel; gemcitabine; sorafenib; lenvatinib; imatinib; sunitinib; regorafenib; mTOR inhibitors and mixtures thereof.
**Item 19:** The combination for use according to any one of items 1 to 18, for use in providing at least an antitumoral activity selected from the group consisting of complement dependent cytotoxicity (CDC), global cytotoxicity, apoptotic activity, impaired spheroid formation, decreased tumor size, tumor invasion and/or metastasis.

### Brief description of the figures

**Figure 1****:** shows the complement dependent cytotoxicity (1 hour incubation) of anti-tumor polyclonal antibody compositions according to the invention, namely PA2.a and PA2.b, in pancreatic adenocarcinoma cell lines.
Figure 1A represents the % of cytotoxicity induced by PA2.a at serial concentrations, ranging from 12.5 µg/mL to 800 µg/mL, in the presence of rabbit complement (1:3 final dilution), for the ASPC1 (top) and PANC-1 (bottom) cell lines (n = 3).
Figure 1B represents the % of cytotoxicity induced by PA2.b at serial concentrations, ranging from 12.5 µg/mL to 800 µg/mL, in the presence of rabbit complement (1:3 final dilution), for each of the following cell lines: ASPC1 (top, first page), MIA-PACA-2 (bottom, first page), PANC-1 (top, second page) and CAPAN-1 (bottom, second page) (*n* = 3).
**Figure 2**: shows the global cytotoxicity (24h incubation) of anti-tumor polyclonal antibody compositions PA2.a and PA2.b on solid cancer cell lines.
Figure 2A represents the % of cytotoxicity observed in the ASPC1 cell line when exposed to serial dilutions of PA2.a, ranging from 12.5 µg/mL to 800 µg/mL, in the presence of rabbit complement (1:3 final dilution).
Figure 2B represents the % of cytotoxicity induced by PA2.b at serial concentrations, ranging from 12.5 µg/mL to 800 µg/mL, in the presence of rabbit complement (1:3 final dilution), for each of the following cell lines: ASPC1 (●), MIA-PACA-2 ( 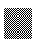 ), PANC-1 ( 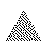 ) and CAPAN-1 ( 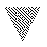 ).
**Figure 3****:** represents the % of cell death in two pancreatic cancer cell lines (ASPC1 (left) and MIA-PACA-2 (right)) exposed to 120 µM of gemcitabine (black) or 100 µg/mL of the anti-tumor polyclonal antibody composition PA2.b, in the presence of rabbit complement (1:3 final dilution) (light grey).
**Figure 4****:** shows the action of the anti-tumor polyclonal antibody composition of the invention, PA2.b, on pancreatic cancer spheroid formation.
ASPC1 cells were untreated (Control PBS, top left picture), treated with 100 µM of gemcitabine (top right picture) or treated with serial concentrations of PA2.b, i.e. 11 µg/mL, 33 µg/mL, 100 µg/mL and 300 µg/mL (bottom pictures).
**Figure 5****:** illustrates the anti-tumor activity of the polyclonal antibody composition PA2.b in an ASPC1 3D culture model. Sphere formation, measured by luminescence, is shown after 10 days of culture. The cells were either left untreated (left) or treated with 100 µg/mL of PA2.b (middle) or 300 µg/mL of PA2.b (right) on days 3 and 7 of culture, all in the presence of rabbit complement (1:6 final dilution).
**Figure 6****:** represents the anti-tumor activity of the polyclonal antibody composition PA2.b in hepatocellular and colorectal cancer cell lines, after 24 hours of incubation. Tumor cells representative of hepatocellular carcinoma (HUH7 ( 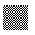 curve) and Hep3b ( 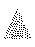 curve)) and colorectal cancer (HCT116 (▼ curve)) were exposed to serial concentrations of PA2.b, ranging from 12.5 µg/mL to 800 µg/mL, in the presence of rabbit complement (1:3 final dilution). The % of cytotoxicity is shown for each of the above-mentioned cell lines.

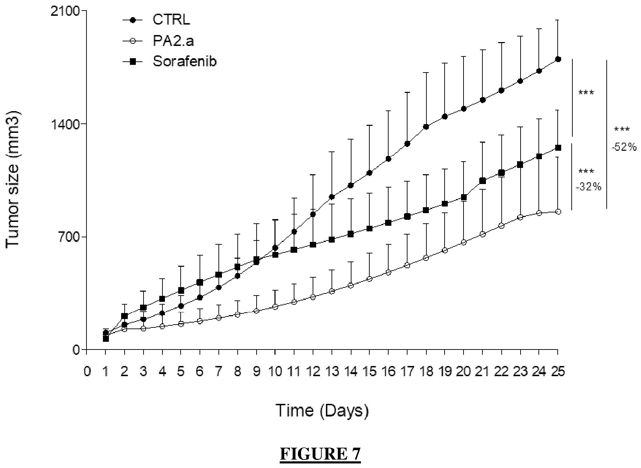

**Figure 7****:** represents the evolution of the tumor size in HUH7 xenograft mice treated with the PA2.a anti-tumor polyclonal antibody composition, or sorafenib.
The figure shows the mean tumor size (in mm³) over time (from day 0 to day 25 of treatment), in the absence of treatment (● curve)), in the presence of PA2.a (o curve) or in the presence of sorafenib (■ curve) (*n* = 10 mice per group).
**Figure 8****:** describes the binding of anti-tumor polyclonal antibody compositions PA2.a and PA2.b to different protein targets.
Figure 8A represents the Z score of PA2.a for the following targets, from left to right: MVP, BCAP31, PEPD, PAXX, ASPH.
Figure 8B represents the Z score of PA2.b for the following targets, from left to right: MVP, SERPINB6, EGFR, CEACAM6 and RNPEP.
**Figure 9****:** describes the binding of the anti-tumor polyclonal antibody composition PA2.a to KRAS^{G12D}.

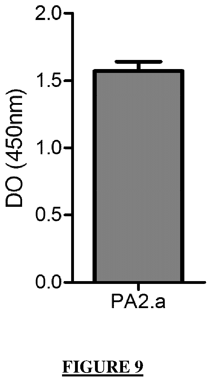

Figure 9 shows the binding of PA2.a to KRAS^{G12D} (in DO (450 nm)).

### Detailed description of the invention

### 1. Definitions

Several definitions are set forth below. Such definitions are meant to encompass grammatical equivalents.

Unless otherwise defined herein, scientific and technical terms used in connection with the present invention shall have the meanings that are commonly understood by those of ordinary skill in the art. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, may provide one of skill with a general dictionary of many of the terms used in this disclosure. In case of conflict, the present specification, including definitions, will control. Exemplary methods and materials are described below, although methods and materials similar or equivalent to those described herein can also be used in the practice or testing of the present invention. Units, prefixes, and symbols are denoted in their Système International des Unités (SI) accepted form. The headings provided herein are not limitations of the various aspects of the disclosure.

All publications and other references mentioned herein are incorporated by reference in their entirety.

It is to be noted that the term "*a*" or "*an*" entity refers to one or more of that entity; for example, "*an antibody,"* is understood to represent one or more antibodies. As such, the terms "*a*" (or "*an*"), "*one or more*" and "*at least one*" can be used interchangeably herein.

Throughout this specification and embodiments, the words "*have*" and "*comprise*," or variations such as "*has*", "*having*", "*comprises*" or *"comprising"* will be understood to imply the inclusion of a stated integer or group of integers but not the exclusion of any other integer or group of integers. The words "*have*" and "*comprise*" or variations such as "*has*", "*having*", "*comprises*" or *"comprising"* will be understood to imply the inclusion of the stated element(s) (such as a composition of matter or a method step) but not the exclusion of any other elements. The term "*consisting of*" implies the inclusion of the stated element(s), to the exclusion of any additional elements. The term "*consisting essentially of*" implies the inclusion of the stated elements, and possibly other element(s) where the other element(s) do not materially affect the basic characteristic(s) of the disclosure. It is understood that the different embodiments of the disclosure using the term "*comprising*" or equivalent cover the embodiments where this term is replaced with "*comprising only*", "*consisting of*" or "*consisting essentially of*".

It is understood that wherever aspects are described herein with the language "*comprising*" otherwise analogous aspects described in terms of "*consisting of*" and/or "*consisting essentially of*" are also provided.

Furthermore, "*and*/*or*" where used herein is to be taken as specific disclosure of each of the two specified features or components with or without the other. Thus, the term "and/or" as used in a phrase such as "A and/or B" herein is intended to include "A and B", "A or B", "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

"*Pharmaceutically*" or "*pharmaceutically acceptable*" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

As used herein, "*pharmaceutically acceptable carriers*" includes any and all solvents, dispersion media, coatings, antibacterial and antifungal agents, and the like that are physiologically compatible. Examples of suitable carriers, diluents and/or excipients include one or more of water, amino acids, saline, phosphate buffered saline, buffer phosphate, acetate, citrate, succinate; amino acids and derivates such as histidine, arginine, glycine, proline, glycylglycine; inorganic salts NaCl, calcium chloride; sugars or polyalcohols such as dextrose, glycerol, ethanol, sucrose, trehalose, mannitol; surfactants such as Polysorbate 80, polysorbate 20, poloxamer 188; and the like, as well as combination thereof. In many cases, it will be preferable to include isotonic agents, such as sugars, polyalcohols, or sodium chloride in the composition, and formulation may also contain an antioxidant such as tryptamine and a stabilizing agent such as Tween 20. Suitable excipients, as well as pharmaceutical formulation requirements, are described in "Remington: The Science & Practice of Pharmacy", which is a reference work in the field.

As used herein the term *"antibody"* have the same meaning and will be used equally in the present description. The term *"antibody"* as used herein refers to isolated or recombinant immunoglobulin molecules and immunologically active portions of immunoglobulin molecules, i.e. molecules that contain an antigen-binding site that immunospecifically binds an antigen, such as proteins MVP, BCAP31, PEPD, PAXX, ASPH, SERPINB6, EGFR, CEACAM6 or RNPEP. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FRs) can participate to the antibody binding site or influence the overall domain structure and hence the combining site. Complementarity Determining Regions, or CDRs, refer to amino acid sequences which together define the binding affinity and specificity of the natural Fv region of a native immunoglobulin binding site. The light and heavy chains of an immunoglobulin each have three CDRs, designated CDR1-L, CDR2-L, CDR3-L and CDR1-H, CDR2-H, CDR3-H, respectively. An antigen-binding site, therefore, typically includes six CDRs, comprising the CDR set from each of a

heavy and a light chain V region. Framework Regions (FRs) refer to amino acid sequences interposed between CDRs.

The term *"antibody"* includes, but is not limited to, monoclonal antibodies, polyclonal antibodies, human antibodies, humanized antibodies, camelid antibodies and chimeric antibodies. The antibodies can be of any isotype/class (e.g., IgG, IgE, IgM, IgD, IgA and IgY), or subclass (e.g., IgG1, IgG2, IgG3, IgG4, IgA1 and IgA2).

In the context of the present disclosure, the term *"antibody",* and in particular the terms "combination of antibodies", specifically includes:
a. an antibody binding a MVP protein (also termed an anti-MVP antibody), and
b.1. an antibody binding to a BCAP31 protein (also termed an anti-BCAP31 antibody), and/or an antibody binding to a PEPD protein (also termed an anti-PEPD antibody), and/or an antibody binding a PAXX protein (also termed an anti-PAXX antibody), and/or an antibody binding to a ASPH protein (also termed an anti-ASPH antibody), or
b.2. an antibody binding a SERPINB6 protein (also termed an anti-SERPINB6 antibody), and/or an antibody binding a EGFR protein (also termed an anti-EGFR antibody), and/or an antibody binding a CEACAM6 protein (also termed an anti-CEACAM6 antibody), and/or an antibody binding a RNPEP protein (also termed an anti-RNPEP antibody).

It is intended that all references to the term *"antibody"* or "*antigen-binding fragment thereof*" as used herein, refer to the antibodies or the antigen-binding fragments thereof as described in the present disclosure.

"*Antigen-binding fragment(s)*" of (conventional) antibodies comprise a portion of an intact antibody, in particular the antigen-binding region or variable region of the intact antibody. Examples of antibody fragments include Fv, Fab, F(ab')2, Fab', dsFv, (dsFv)2, scFv, sc(Fv)2, diabodies, bispecific and multispecific antibodies formed from antibody fragments. A fragment of a conventional antibody may also be a single domain antibody, such as a heavy chain antibody or VHH.

The term "*Fab*" denotes an antibody fragment having a molecular weight of about 50,000 and antigen-binding activity, in which about a half of the N-terminal side of the heavy chain and the entire light chain are bound together through a disulfide bond. It is usually obtained among fragments by treating IgG with a protease, papain.

The term "*F(ab')2*" refers to an antibody fragment having a molecular weight of about 100,000 and antigen-binding activity, which is slightly larger than 2 identical Fab fragments bound via a disulfide bond of the hinge region. It is usually obtained among fragments by treating IgG with a protease, pepsin.

The term "*Fab'*" refers to an antibody fragment having a molecular weight of about 50,000 and antigen-binding activity, which is obtained by cutting a disulfide bond of the hinge region of the F(ab')2.

A single chain Fv ("*scFv*") polypeptide is a covalently linked VH::VL heterodimer which is usually expressed from a gene fusion including VH and VL encoding genes linked by a peptide-encoding linker. The human scFv fragment of the disclosure includes CDRs that are held in appropriate conformation, in particular by using gene recombination techniques. Divalent and multivalent antibody fragments can form either spontaneously by association of monovalent scFvs, or can be generated by coupling monovalent scFvs by a peptide linker, such as divalent sc(Fv)2. "dsFv" is a VH::VL heterodimer stabilized by a disulphide bond. "(dsFv)2" denotes two dsFv coupled by a peptide linker.

The term "bispecific antibody" or "BsAb" denotes an antibody which combines the antigen-binding sites of two antibodies within a single molecule. Thus, BsAbs are able to bind two different antigens simultaneously. Genetic engineering has been used with increasing frequency to design, modify, and produce antibodies or antibody derivatives with a desired set of binding properties and effector functions as described for instance in EP 2 050 764 A1.

The term "*multispecific antibody*" denotes an antibody which combines the antigen-binding sites of two or more antibodies within a single molecule.

The term "*diabodies*" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains of the same chain, the domains are forced to pair with the complementary domains of another chain and create two antigen-binding sites.

Antibodies of compositions according to the invention may be produced by any technique known in the art, such as, without limitation, any chemical, biological, genetic or enzymatic technique, either alone or in combination. The antibodies of the present invention may comprise polyclonal antibodies. Methods of preparing polyclonal antibodies are known to the skilled artisan (Harlow et al., Antibodies: a Laboratory Manual, Cold Spring Harbor Laboratory Press, 2nd ed. (1988)).

A "*combination of antibodies, or antigen-binding fragments thereof*" may in particular be a polyclonal antibody composition or a combination of monoclonal antibodies, and may preferably be a polyclonal antibody composition.

By *"polyclonal antibodies", "polyclonal antibody"* or *"polyclonal antibody composition"* as used herein in an interchangeable manner is meant a mixture of antibodies recognizing different epitopes of a given antigen, or even different epitopes of different antigens expressed by a given cell or group of cells. Polyclonal antibodies encompass those which are contained in, or alternatively which are derived from, body fluids, especially serum or plasma from a non-human mammal organism, in particular from a pig or a rabbit. A polyclonal antibody composition of the invention is different from a composition containing more than one monoclonal antibody.

The term *"monoclonal antibody"* or "*mAb*" as used herein refers to an antibody molecule of a single amino acid sequence, which is directed against a specific antigen, and is not to be construed as requiring production of the antibody by any particular method. A monoclonal antibody may be produced by a single clone of B cells or hybridoma, but may also be recombinant, i.e., produced by protein engineering.

The term *"humanized antibody"* refers to an antibody which is wholly or partially of non-human origin, and which has been modified to replace certain amino acids, in particular in the framework regions of the VH and VL domains, in order to avoid or minimize an immune response in humans. The constant domains of a humanized antibody are most of the time human CH and CL domains.

The term *"recombinant"* as applied to an antibody, or an antigen-binding fragment thereof, a nucleic acid sequence, an expression vector or a host cell means that those are the products of various combinations of *in vitro* cloning, restriction, ligation steps, and other genetic engineering procedures.

In the context of the present invention, the term *"antibody"* in particular encompasses an antibody from a pig or a rabbit, and more particularly an antibody from a pig or a rabbit, the said antibody being devoid of at least one antigenic determinant selected from (i) N-glycolylneuraminic acid (Neu5Gc) and (ii) α-1,3-galactose, and in particular is devoid of the two antigenic determinants N-glycolylneuraminic acid (Neu5Gc) and α-1,3-galactose; and which further preferably comprises at least one sugar moiety distinct from the antigenic determinants (i) N-glycolylneuraminic acid (Neu5Gc) and/or (ii) α-1,3-galactose.

A combination of antibodies for use according to the invention, and more particularly a polyclonal antibody composition for use according to the invention, as understood herein, is in particular a polyclonal antibody composition that is obtainable by immunization of an animal with pancreatic tumor cell antigens comprising KRAS mutated antigens. In particular, the animal is a pig or a rabbit, and in particular is a pig or a rabbit lacking at least one gene selected in a group comprising (i) a gene encoding a functional cytidine-5'-monophosphate N-acetyl neuraminic acid hydrolase (CMAH) and (ii) a gene encoding a functional α-(1,3)-galactosyltransferase, and more particularly lacking both (i) a gene encoding a functional cytidine-5'-monophosphate N-acetyl neuraminic acid hydrolase (CMAH) and (ii) a gene encoding a functional α-(1,3)-galactosyltransferase.

The term *"antigen"* as used in the present disclosure refers to a molecule or a portion of a molecule capable of being bound by one or more antibodies. An antigen can have one or more than one epitope. For instance, in the context of the disclosure, an antigen is in particular selected from the group consisting of proteins MVP, BCAP31, PEPD, PAXX, ASPH, SERPINB6, EGFR, CEACAM6 and RNPEP.

The term *"affinity",* as used herein, means the strength of the binding of an antibody to an epitope presented on an antigen. The affinity of an antibody is given by the dissociation constant KD, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Ka is defined by 1/Kd. Methods for determining the affinity of Abs can be found in, for example, Harlow *et al*., Cold Spring Harbor Laboratory Press, Cold Spring Harbor, N.Y. (1988), Coligan et al., Current Protocols in Immunology, Greene Publishing Assoc. and Wiley Interscience, N.Y. (1992, 1993), or Müller, Methods Enzymol. 92:589-601 (1983), which references are entirely incorporated herein by reference. A preferred and standard method well known in the art for determining the affinity of mAbs is the measurement of surface plasmon resonance by using the Biacore instruments (Laure et al., Curr Protoc Protein Sci. Sep;Chapter 19:Unit 19.13 (2006)). For multimeric antigens, such as virus capsids, to which both antigen-binding sites of an antibody can bind simultaneously, Ka and KD values determined by such standard methods measure the functional affinity, or avidity of the interaction. Illustratively, an antibody is considered to bind an antigen when a functional binding affinity (KD), preferably measured by surface plasmon resonance, is of 10-8 mol/l (M) or less, preferably 10-9 M to 10-12 M.

By *"IgG"* as used herein is meant a polypeptide belonging to the class of antibodies that are substantially encoded by a recognized immunoglobulin gamma gene. In humans, IgG comprises the subclasses or isotypes IgG1, IgG2, IgG3, and IgG4. In mice, IgG comprises IgG1, IgG2a, IgG2b, IgG3. In pigs and rabbits, immunoglobulins comprise the classes or isotypes IgM, IgG, IgE and IgA antibodies and the IgG isotype comprise 11 subclasses (Butler et al., Developmental and Comparative Immunology 30:199-221 (2006) and Butler et al., Developmental and Comparative Immunology 33:321-333 (2009)). Full-length IgGs consist of two identical pairs of two immunoglobulin chains, each pair having one light and one heavy chain, each light chain comprising immunoglobulin domains VL and CL, and each heavy chain comprising immunoglobulin domains VH, Cγ1 (also called CH1), Cγ2 (also called CH2), and Cγ3 (also called CH3).

By *"antigenic determinant"* (or epitope), as applied herein in particular to pig or rabbit antibodies, as used herein is meant a structural component of an antigenic molecule, which includes an antigenic protein and an antigenic carbohydrate, responsible for its specific interaction with antibody molecules elicited by the same or related antigen. By extension, the term "antigenic determinant", as applied herein to pig or rabbit antibodies, is also used collectively herein for an antigenic molecule comprising a plurality of epitopes, including conformational motives in which the sugar moiety is needed but represent only part of the epitope, susceptible to be recognized by antibody molecules elicited by the same or related antigen. Illustratively, the antigenic molecule N-glycolylneuraminic acid (Neu5Gc) may be called herein an "antigenic determinant", although the said antigenic molecule may exhibit more than one epitope recognized by antibodies elicited with Neu5Gc or with Neu5Gc containing molecules.

By *"conventional polyclonal antibodies",* as used herein, is meant polyclonal antibodies, and in particular pig or rabbit polyclonal antibodies, that are not devoid of the antigenic determinants N-glycolylneuraminic acid (Neu5Gc) and α-1,3-galactose.In this regard, it may be notably cited the products commercialized under the name Thymoglobulin, Grafalon, Atgam or p-ALG^{®}.

The term *"cancer"* is herein used in its traditional sense and refers to a cell or group of cells displaying uncontrolled growth, invasion upon adjacent tissues.

Examples of cancers more particularly considered in the present invention are provided further. Cancers may in particular refer herein to a cancer selected from the group consisting of myeloma; melanoma; breast cancer, in particular triple negative breast cancer; prostate cancer; primary and metastatic colorectal cancer, in particular colon cancer or metastatic colon cancer; lung cancer, in particular non small cell lung cancer (NSCLC) or small cell lung cancer (SCLC); liver cancer, in particular hepatocarcinoma or cholangiocarcinoma; primary and metastatic pancreas cancer, in particular pancreatic adenocarcinoma; lymphoma, in particular B-cell or T-cell lymphoma, more particularly T-cell lymphoma; gastric cancer; head and neck cancer; ovarian cancer; sarcoma; acute and chronic leukemia; osteosarcoma; anal cancer; testicular cancer; uterus cancer; thyroid cancer; cancer of the central nervous system; gastrointestinal stromal cancer; epidermal carcinoma and oesophageal cancer.

Cancers also considered in the present invention are cancers comprising cancer cells harboring at least one KRAS mutation, such as in particular at least one KRAS mutation selected from the group consisting of KRAS^{G12A}, KRAS^{G12C}, KRAS^{G12D}, KRAS^{G12S}, KRAS^{G12V}, KRAS^{G13C}, KRAS^{G13D} and KRAS^{G12R} mutation(s), more particularly KRAS^{G12D}, KRAS^{G12C}, and KRAS^{G12V} mutation(s).

By a *"therapeutically effective amount"* of a combination of antibodies used according to the invention is meant a sufficient amount of the antibodies to obtain the desired effect as presented in the present text, and in particular for treating a human subject affected with a cancer, that is, a human subject in need thereof. It will be understood, however, that the total daily usage of the combination of antibodies implemented according to the invention will be decided by the attending physician within the scope of sound medical judgment. The specific therapeutically effective dose level for any particular patient will depend upon a variety of factors including the nature of the cancer being treated; the activity of the specific antibodies employed; the specific composition employed; the age, body weight, general health, sex and diet of the patient; the time of administration, route of administration, and rate of excretion of the specific antibodies employed; the duration of the treatment; drugs used in combination or coincidental with the specific antibodies employed; and like factors well known in the medical arts. For example, it is well known within the skill of the art to start doses of the compound at levels lower than those required to achieve the desired therapeutic effect and to gradually increase the dosage until the desired effect is achieved.

The terms *"treatment", "treating"* or *"therapy"* refers to administering an active agent with the purpose to cure, heal, alleviate, relieve, alter, remedy, ameliorate, improve, or affect a condition (i.e., a cancer), the symptoms of the cancer, or to prevent or delay the onset of the symptoms, complications, biochemical indicia of a disease, or otherwise arrest or inhibit further development of the cancer. In particular, in the case of the present invention, treating a cancer includes providing an antitumoral activity. In particular, the antitumoral activity comprises cytotoxicity complement dependent (CDC), global cytotoxicity, apoptotic activity, impaired spheroid formation, decreased tumor size, tumor invasion and/or metastasis.

The terms *"wild type animal"* or *"WT animal"* come herein in opposition with a genetically altered animal. For example, by "wild type pig or rabbit" is meant a pig or rabbit which is not lacking at least one gene selected in a group comprising (i) a gene encoding a functional cytidine-5'-monophosphate N-acetyl neuraminic acid hydrolase (CMAH) and (ii) a gene encoding a functional α-(1,3)-galactosyltransferase.

As an appropriate method for obtaining polyclonal antibodies which may be present in antibody combinations implemented according to the present invention, may notably be cited the method of fractionated precipitation with ethanol, with ammonium sulfate, with rivanol, with polyethylene glycol or with caprylic acid; the method by passage through ion exchange columns; other methods can involve affinity columns on protein A or G. The antibodies obtained can then be subjected to conventional treatments for their intravenous administration, for example by enzymatic cleavage treatments with plasmin, papain or pepsin. In this regard, may be more particularly cited the protocol implemented in example 3 of EP 0 335 804, which implements an ion exchange chromatography on DEAE cellulose.

Such antibodies can for example be generated through immunization of a non-human animal, in particular a non-human mammal, according to methods well known to the man skilled in the art. The non-human mammal may be selected from the group consisting of rodents, such as mice, rats, guinea pigs and hamsters; lagomorphs, such as rabbits; ferrets; felines, such as cats; canines, such as dogs; goats; sheep; bovines, such as cows; swines, such as pigs and hogs; camelids; horses; and non-human primates. The non-human mammal may more particularly be a pig or a rabbit. Accordingly, by *"anti-cancer pAb from pig origin"* for example, it is meant that the polyclonal antibodies have been obtained through immunization of a pig with a combination of proteins of interest or with a cell naturally expressing, or genetically altered in order to express on its surface the proteins of interest against which it is desired to obtained polyclonal antibodies. See Reynard et al., pLoS One 11(6):e0156775 (2016); Schieferdecker et al., Oncotarget Oct 11; 7(41):67061-67070 (2016) and Zhang et al., Sci Rep 4, 4984 (2014) (DOI: 10.1038/srep04984), incorporated herein by reference.

The terms *"polypeptide"* and *"protein"* are used interchangeably herein to refer to a sequence of amino acid residues. The terms apply to amino acid sequences in which one or more amino acid residue is an artificial chemical mimetic of a corresponding naturally occurring amino acid, as well as to naturally occurring amino acid sequences and non-naturally occurring amino acid sequence.

### 2. Antibody combinations implemented according to the invention

As mentioned above, the present invention relates to the implementation of a combination of antibodies, or antigen-binding fragments thereof, for use in the treatment of a cancer in a human subject in need thereof.

Such combination comprises at least a first antibody, or an antigen-binding fragment thereof, specifically binding to MVP (Major vault protein).

In addition to this first antibody or antigen-binding fragment thereof, a combination of antibodies, or antigen-binding fragments thereof, implemented according to the invention further comprises:
(i) at least an antibody (a), or an antigen-binding fragment thereof, specifically binding to at least an antigen selected from the group consisting of BCAP31 (B cell receptor-associated protein 31), PEPD (Peptidase D), PAXX (Paralog of x-ray repair cross-complementing protein 4 (XRCC4) and XRCC4-like factor (XLF)), ASPH (Aspartate β-hydroxylase) and mutated KRAS (Kirsten rat sarcoma viral oncogene homologue) subtype KRAS^{G12D}; and/or, and in particular or,
(ii) at least an antibody (b), or an antigen-binding fragment thereof, specifically binding to at least an antigen selected from the group consisting of SERPINB6 (Serine protease inhibitor B6), EGFR (Epidermal growth factor receptor), CEACAM6 (Carcinoembryonic antigen-related cell adhesion molecule 6) and RNPEP (Arginyl aminopeptidase (aminopeptidase B)).

Each one of the antibodies present in a combination for use according to the invention may, independently from the others, be a monoclonal antibody, a polyclonal antibody or an antigen-binding fragment thereof. As defined above, said antigen-binding fragment may independently be selected from the group consisting of Fv, Fab, F(ab')2, Fab', dsFv, (dsFv)2, scFv, sc(Fv)2, or diabodies.

According to an embodiment, the combination of antibodies for use according to the invention may comprise more than one, in particular more than two, more particularly more than three, in particular more than four, and in particular five, different proteins among the group consisting of BCAP31, PEPD, PAXX, ASPH and mutated KRAS subtype KRAS^{G12D}. The antibodies present in a combination of antibodies for use according to the invention may accordingly target, in addition to MVP, at least 2 proteins, target 3 proteins, target at least 3 proteins, target 4 proteins, target at least 4 proteins or target 5 proteins from the group consisting of BCAP31, PEPD, PAXX, ASPH and mutated KRAS subtype KRAS^{G12D},

In particular, a combination of antibodies for use according to the invention comprises antibodies targeting 2, 3, 4 or 5, more particularly 3, 4 or 5, even more particularly 4 or 5, in particular 5, proteins selected from the group consisting of BCAP31, PEPD, PAXX, ASPH and mutated KRAS subtype KRAS^{G12D},

According to an embodiment, the combination of antibodies for use according to the invention may comprise more than one, in particular more than two, more particularly more than three, in particular four, different proteins among the group consisting of SERPINB6, EGFR, CEACAM6 and RNPEP. The antibodies present in a combination of antibodies for use according to the invention may accordingly target, in addition to MVP, at least 2 proteins, target 3 proteins, target at least 3 proteins or target 4 proteins from the group consisting of SERPINB6, EGFR, CEACAM6 and RNPEP.

In particular, a combination of antibodies for use according to the invention comprises antibodies targeting 2, 3 or 4, more particularly 3 or 4, even more particularly 4, proteins selected from the group consisting of SERPINB6, EGFR, CEACAM6 and RNPEP.

Some or all of the antibodies of an antibody combination for its use according to the invention may be devoid of at least one antigenic determinant selected from (i) N-glycolylneuraminic acid (Neu5Gc) and (ii) α-1,3-galactose. Some or all of the said antibodies for use according to the invention may in particular be devoid of the two antigenic determinants N-glycolylneuraminic acid (Neu5Gc) and α-1,3-galactose.

A method allowing to identify or characterize such antibodies falls within the general knowledge of a man skilled in the art. A method that may be used by the one skilled in the art for identifying or characterizing antibodies according to the invention includes an Enzyme-linked immunosorbent assay (ELISA) wherein, for example, anti-Neu5Gc antibodies and anti-Gal antibodies are used as detection molecules.

The antibodies from the antibody combination implemented according to the invention may be immunoglobulin G antibodies.

Porcine IgG structure and genetics have for example been described in literature (Butler *et al*., 2009 - DOI: 10.1007/s00251-009-0356-0). Eleven isotypes called IgG1a, IgG1b, IgG2a, IgG2b, IgG3, IgG4a, IgG4b, IgG5a, IgG5b, IgG6a, IgG6b have been proposed based on analysis of the genomic IgH locus sequences. According to the available knowledge on relative abundance of pig IgG subclasses and Fc domains affinity for Protein A (Butler *et al*., 2009 - DOI: 10.1007/s00251-009-0356-0), during a conventional purification, the relative composition of the IgG isotypes in pig DKO polyAb is estimated to be >80% pig IgG1a/b, of 11% IgG2a/b, of 5.5% IgG3, of 3% IgG4a/b, the remaining fraction being other isotypes (IgG5-6). No detectable IgM or IgA isotype is present in pig DKO IgG polyclonal antibody preparation after purification using Protein A chromatography.

Protein MVP (Major vault protein) is a structural component of vault ribonucleoprotein particles, which are involved in various cellular processes, including drug resistance, signal transduction, and possibly intracellular transport.

The amino acid sequence of MVP has for reference UNIPROT Q14764.

Antibodies able to specifically target MVP are well known in the art. Can for example be mentioned monoclonal anti-MVP WH0009961M1 (commercialized by Merck),

or polyclonal antibody anti-MVP PA5-80836 (commercialized by Thermo Fischer Scientific).

Protein BCAP31 (B cell receptor-associated protein 31) is a transmembrane protein located in the endoplasmic reticulum and is involved in the trafficking and processing of membrane proteins, including its role in apoptosis and immune signaling pathways.

The amino acid sequence of BCAP31 has for reference UNIPROT P51572.

Antibodies able to specifically target BCAP31 are well known in the art. Can for example be mentioned monoclonal antibody anti-BCAP31 RAB07584 (commercialized by Abnova) or polyclonal antibody anti-BCAP31 201968-T42 (commercialized by Sino Biological, Inc).

Protein PEPD (Peptidase D) is an enzyme involved in the breakdown of dipeptides, particularly proline-containing peptides, and plays a key role in collagen metabolism and maintaining amino acid balance.

The amino acid sequence of PEPD has for reference UNIPROT P12955.

Antibodies able to specifically target PEPD are well known in the art. Can for example be mentioned monoclonal antibody anti-PEPD sc-100708 (commercialized by Santa Cruz Biotechnology, Inc.) or polyclonal antibody anti-PEPD PA5-84887 (commercialized by Thermo Fischer scientific).

Protein PAXX (Paralog of x-ray repair cross-complementing protein 4 (XRCC4) and XRCC4-like factor (XLF)) is a component of the non-homologous end joining (NHEJ) pathway, crucial for the repair of DNA double-strand breaks, thus maintaining genomic stability.

The amino acid sequence of PAXX has for reference UNIPROT Q9BUH6.

Antibodies able to specifically target PAXX are well known in the art. Can for example be mentioned monoclonal antibody anti-PAXX 92448S (commercialized by Cell Signaling Technology) or polyclonal antibody anti-PAXX NBP3-30539 (commercialized by Novus Biological).

Protein ASPH (Aspartate β-hydroxylase) is an enzyme that catalyzes the hydroxylation of aspartic acid residues in certain proteins, playing a role in protein post-translational modification and is implicated in cancer metastasis and tumor progression.

The amino acid sequence of ASPH has for reference UNIPROT Q12797.

Antibodies able to specifically target ASPH are well known in the art. Can for example be mentioned mentioned monoclonal antibody anti-ASPH H00000444-M09 (commercialized by Abnova) or polyclonal antibody anti-ASPH 144-62656 (commercialized by RayBiotech).

Mutated protein KRAS (Kirsten rat sarcoma viral oncogene homologue) subtype KRAS^{G12D} is a common oncogenic mutation where glycine at position 12 is replaced by aspartic acid.

The amino acid sequence of mutated KRAS subtype KRAS^{G12D} has for reference UNIPROT P01116.

Antibodies able to specifically target mutated KRAS subtype KRAS^{G12D} are well known in the art. Can for example be mentioned monoclonal antibody anti-KRAS G12D GTX639096 (commercialized by GeneTex) or polyclonal antibody anti-KRAS G12Dab221163 (commercialized by Abcam).

Protein SERPINB6 (Serine protease inhibitor B6) is an intracellular serine protease inhibitor that protects against protease-mediated damage, particularly in the inner ear, and mutations in this protein are associated with non-syndromic hearing loss.

The amino acid sequence of SERPINB6 has for reference UNIPROT P35237.

Antibodies able to specifically target SERPINB6 are well known in the art. Can for example be mentioned monoclonal antibody anti-SerpinB6 sc-398463 (commercialized by Santa Cruz Biotechnology, Inc) or polyclonal antibody anti-SerpinB6 PA5-82387 (commercialized by Thermo Fischer Scientific).

Protein EGFR (Epidermal growth factor receptor) is a transmembrane receptor tyrosine kinase involved in the regulation of cell growth, survival, and differentiation.

The amino acid sequence of EGFR has for reference UNIPROT P00533.

Antibodies able to specifically target EGFR are well known in the art. Can for example be mentioned monoclonal antibody anti-EGFR TA386419 (commercialized by Origene) or polyclonal antibody anti-EGFR bs-34018R (commercialized by Bioss Inc).

Protein CEACAM6 (Carcinoembryonic antigen-related cell adhesion molecule 6) is a cell surface glycoprotein involved in cell adhesion and intracellular signaling.

The amino acid sequence of CEACAM6 has for reference UNIPROT P40199.

Antibodies able to specifically target CEACAM6 are well known in the art. Can for example be mentioned monoclonal antibody anti-CEACAM6 85102S (commercialized by Cell Signalling Technology) or polyclonal antibody anti-CEACAM6 10823-RP01 (commercialized by Sino Biological, Inc.).

Protein RNPEP (Arginyl aminopeptidase (aminopeptidase B)) is an enzyme that catalyzes the removal of N-terminal arginine residues from peptides and proteins and is involved in the regulation of blood pressure and the processing of bioactive peptides such as angiotensin.

The amino acid sequence of RNPEP has for reference UNIPROT Q9H4A4.

Antibodies able to specifically target RNPEP are well known in the art. Can for example be mentioned monoclonal antibody anti-RNPEP PA5-70310 (commercialized by Thermo Fischer Scientific) or polyclonal antibody anti-RNPEP ABIN7443036 (commercialized by antibodies-online).

In an embodiment according to the invention, a combination of antibodies, or of antigen-binding fragment thereof, implemented according to the invention may comprise:
(i) at least a first antibody, or an antigen-binding fragment thereof, specifically binding to MVP; and
(ii) a/at least an antibody, or an antigen-binding fragment thereof, specifically binding to BCAP31, and
   b/ at least an antibody, or an antigen-binding fragment thereof, specifically binding to PEPD, and
   c/ at least an antibody, or an antigen-binding fragment thereof, specifically binding to PAXX, and
   d/ at least an antibody, or an antigen-binding fragment thereof, specifically binding to ASPH, and
   e/ at least an antibody, or an antigen-binding fragment thereof, specifically binding to mutated KRAS subtype KRAS^{G12D},

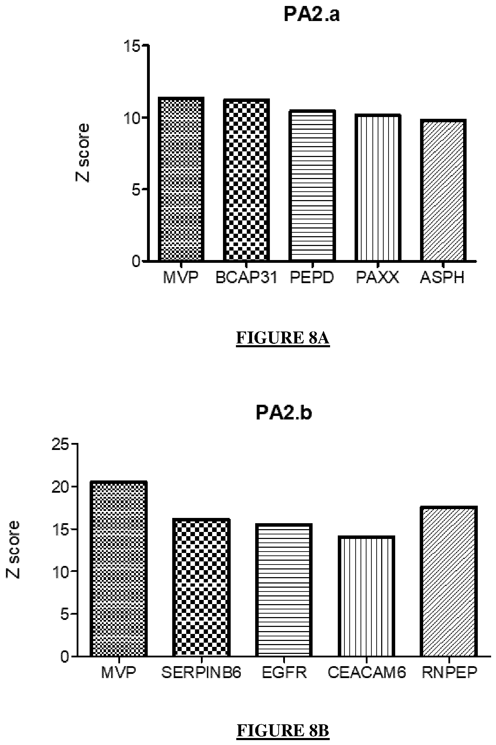

An example of such a combination is illustrated in the examples of the present specification under the denomination "PA2.a", as can be seen from Figure 8A.

In an embodiment according to the invention, a combination of antibodies, or of antigen-binding fragment thereof, implemented according to the invention may comprise:
(i) at least a first antibody, or an antigen-binding fragment thereof, specifically binding to MVP; and
(ii) a/ at least an antibody, or an antigen-binding fragment thereof, specifically binding to SERPINB6, and
   b/ at least an antibody, or an antigen-binding fragment thereof, specifically binding to EGFR, and
   c/ at least an antibody, or an antigen-binding fragment thereof, specifically binding to CEACAM6, and
   d/ at least an antibody, or an antigen-binding fragment thereof, specifically binding to RNPEP.

An example of such a combination is illustrated in the examples of the present specification under the denomination "PA2.b", as can be seen from Figure 8B.

As indicated previously, in all of these embodiments, the antibodies of the combinations, in particular the polyclonal antibodies of the combinations, may, independently from the others, be devoid of at least one antigenic determinant selected from (i) N-glycolylneuraminic acid (Neu5Gc) and (ii) α-1,3-galactose, and may more particularly be devoid of the two antigenic determinants N-glycolylneuraminic acid (Neu5Gc) and α-1,3-galactose.

In a particular embodiment, in all of the above-mentioned embodiments, the antibodies of the combinations, in particular the polyclonal antibodies, are devoid of at least one antigenic determinant selected from (i) N-glycolylneuraminic acid (Neu5Gc) and (ii) α-1,3-galactose, and are more particularly devoid of the two antigenic determinants N-glycolylneuraminic acid (Neu5Gc) and α-1,3-galactose.

The antibodies of a combination for use according to the invention, and in particular polyclonal antibodies for use according to the invention, may be of any origin, such as from a non-human mammal or synthetic, monoclonal or polyclonal. Such non-human mammal may be selected from the group consisting of rodents, such as mice, rats, guinea pigs and hamsters; lagomorphs, such as rabbits; ferrets; felines, such as cats; canines, such as dogs; goats; sheep; bovines, such as cows; swines, such as pigs and hogs; camelids; horses; and non-human primates. The antibodies of a combination for use according to the invention, and in particular polyclonal antibodies for use according to the invention, may in particular be of swine or lagomorph origin, and in particular of pig or rabbit origin.

The antibodies of a combination for use according to the invention, and in particular polyclonal antibodies for use according to the invention, are preferably from a pig

or a rabbit, and in particular from a pig or a rabbit lacking at least one gene selected in a group comprising (i) a gene encoding a functional cytidine-5'-monophosphate N-acetyl neuraminic acid hydrolase (CMAH) and (ii) a gene encoding a functional α-(1,3)-galactosyltransferase, and more particularly lacking both (i) a gene encoding a functional cytidine-5'-monophosphate N-acetyl neuraminic acid hydrolase (CMAH) and (ii) a gene encoding a functional α-(1,3)-galactosyltransferase.

In particular, the combination for use according to the present invention may be a combination of monoclonal antibodies or a polyclonal antibody composition and is preferably a polyclonal antibody composition.

In a particular embodiment, the combination for use according to the present invention is included in a pharmaceutical composition further comprising a pharmaceutically acceptable carrier. Pharmaceutically acceptable carriers are defined further above.

The pharmaceutical composition may be in liquid form.

The pharmaceutical composition may be in a solid form, which includes a lyophilized form.

The pharmaceutical composition may be formulated according to standard methods such as those described in Remington: The Science and Practice of Pharmacy (Lippincott Williams & Wilkins; Twenty first Edition, 2005).

The pharmaceutical composition may further comprise at least one additional anticancer drug different from the antibodies of the combination of antibodies.

The terms *"combination of antibodies"* are also used herein to designate "*a combination of antibodies, or antigen-binding fragments thereof'.*

The additional anticancer drug may for example be selected from the group consisting of monoclonal antibodies, in particular selected from the group consisting of anti-CD19, anti-CD20, anti-CD30, anti-CD137, anti-CTLA4, anti-TIM-3, anti-B7-H3, anti-CD123, anti-CD134, anti-CD154, anti-LAG-3, anti-CD227, anti-BTNA3, anti-CD39, anti-CD73, anti-CD115, anti-CD47, anti-SIRP alpha, anti-SIRP gamma, anti-CD28, anti-NCR, anti-NKp46, anti-NKp30, anti-NKp44, anti-NKG2D, anti-PD1, anti-PDL1, mogamulizumab, obinutuzumab, polatuzumab vedotin, Yttrium Y 90-ibritumomab tiuxetan, mosunetuzumab, cetuximab, atezolizumab/bevacizumab, anti-VEGF antibodies, anti-DNAM-1 monoclonal antibodies and mixtures thereof.

The pharmaceutical composition may further comprise at least one chemotherapy treatment.

Chemotherapy treatments may be selected among those known in the art. In particular, one skilled in the art will know to adapt the choice of the chemotherapy according to the cancer that is to be treated. In a particular embodiment, the chemotherapy treatment is selected from the group consisting of a chemotherapy regimen consisting of Cyclophosphamide, Hydroxydaunorubicin, Oncovin and Prednisone (CHOP); a chemotherapy regimen consisting of Cyclophosphamide, Hydroxydaunorubicin, Oncovin, Etoposide and Prednisone (CHOEP); HDAC inhibitors; ibrutinib; acalabrutinib; zanubrutinib; copanlisib; venetoclax; folfirinox; cisplatin; doxorubicin; irinotecan; liposomal irinotecan; oxaliplatin; Nap paclitaxel; paclitaxel; gemcitabine; sorafenib; lenvatinib; imatinib; sunitinib; regorafenib; mTOR inhibitors and mixtures thereof.

Dosages may range from 0.001 to 100 mg or more of the combination for use according to the invention as defined herein per kg of body weight (mg/kg) or greater, for example 0.1, 1.0, 10, or 50 mg/kg of body weight, with 1 to 20 mg/kg being preferred. The dosage and frequency of administration may be adapted as previously detailed.

Also, any injection of a combination for use according to the invention may be followed by any usual procedure to prevent and/or avoid anaphylactic reaction.

Besides, the injection of a combination or composition implemented according to the invention may be performed through a large peripheral access or, if possible, through a central catheter.

As is known in the art, adjustments for protein degradation, systemic versus localized delivery, as well as the age, body weight, general health, sex, diet, time of administration, possible allergy, drug interaction and the severity of the condition may be necessary, and is easily determined with routine experimentation by those skilled in the art.

Administration of the combination or composition for use of the invention may be done in a variety of ways, including, but not limited to, orally, subcutaneously, intravenously, parenterally, intranasally, intrarespiratory (such as nebulization or intratracheal spray), intraortically, intraocularly, rectally, vaginally, transdermally, topically (e.g., gels), intraperitoneally, intramuscularly, intrapulmonary or intrathecally.

Administration of the combination or composition of the invention may be done by following the Besredka method.

A combination or composition according to the invention may in particular be in a form suitable for administration by intravenous route.

### 3. Implementation of a combination of antibodies, or antigen-binding fragments thereof, or a composition, according to the invention

A combination of antibodies, or antigen-binding fragments thereof, or a composition comprising it, implemented according to the invention is used in a therapeutically effective amount.

As previously indicated, a combination of antibodies or composition thereof is for use in the treatment of a cancer in a human subject.

The cancer may in particular be selected from the group consisting myeloma; melanoma; breast cancer, in particular triple negative breast cancer; prostate cancer; primary and metastatic colorectal cancer, in particular colon cancer or metastatic colon cancer; lung cancer, in particular non small cell lung cancer (NSCLC) or small cell lung cancer (SCLC); liver cancer, in particular hepatocarcinoma or cholangiocarcinoma; primary and metastatic pancreas cancer, in particular pancreatic adenocarcinoma; lymphoma, in particular B-cell or T-cell lymphoma, more particularly T-cell lymphoma; gastric cancer; head and neck cancer; ovarian cancer; sarcoma; acute and chronic leukemia; osteosarcoma; anal cancer; testicular cancer; uterus cancer; thyroid cancer; cancer of the central nervous system; gastrointestinal stromal cancer; epidermal carcinoma and oesophageal cancer. The cancer may in particular be selected from the group consisting of pancreas cancer, in particular pancreatic adenocarcinoma; liver cancer, in particular hepatocarcinoma or cholangiocarcinoma; colorectal cancer, in particular metastatic colon cancer; and lung cancer, in particular non small cell lung cancer (NSCLC).

In particular, the cancer is selected from the group consisting of pancreas cancer, in particular pancreatic adenocarcinoma; liver cancer, in particular hepatocellular carcinoma; and colorectal cancer, in particular colon cancer, more particularly metastatic colon cancer.

In a particular embodiment, a combination for use according to the invention is such that it comprises:
(i) at least a first antibody, or an antigen-binding fragment thereof, specifically binding to MVP; and
(ii) a/at least an antibody, or an antigen-binding fragment thereof, specifically binding to BCAP31, and
   b/ at least an antibody, or an antigen-binding fragment thereof, specifically binding to PEPD, and
   c/ at least an antibody, or an antigen-binding fragment thereof, specifically binding to PAXX, and
   d/ at least an antibody, or an antigen-binding fragment thereof, specifically binding to ASPH, and
   e/ at least an antibody, or an antigen-binding fragment thereof, specifically binding to mutated KRAS subtype KRAS^{G12D};

with the cancer being selected from the group consisting of myeloma; melanoma; breast cancer, in particular triple negative breast cancer; prostate cancer; primary and metastatic colorectal cancer, in particular colon cancer or metastatic colon cancer; lung cancer, in particular non small cell lung cancer (NSCLC) or small cell lung cancer (SCLC); liver cancer, in particular hepatocarcinoma or cholangiocarcinoma; primary and metastatic pancreas cancer, in particular pancreatic adenocarcinoma; lymphoma, in particular B-cell or T-cell lymphoma, more particularly T-cell lymphoma; gastric cancer; head and neck cancer; ovarian cancer; sarcoma; acute and chronic leukemia; osteosarcoma; anal cancer; testicular cancer; uterus cancer; thyroid cancer; cancer of the central nervous system; gastrointestinal stromal cancer; epidermal carcinoma and oesophageal cancer. In particular, the cancer may be selected from the group consisting of pancreas cancer, in particular pancreatic adenocarcinoma; liver cancer, in particular hepatocarcinoma or cholangiocarcinoma; colorectal cancer, in particular metastatic colon cancer; and lung cancer, in particular non small cell lung cancer (NSCLC).

In particular, the cancer is selected from the group consisting of pancreas cancer, in particular pancreatic adenocarcinoma, and liver cancer, in particular hepatocellular carcinoma.

In a particular embodiment, a combination for use according to the invention is such that it comprises:
(i) at least a first antibody, or an antigen-binding fragment thereof, specifically binding to MVP; and
(ii) a/ at least an antibody, or an antigen-binding fragment thereof, specifically binding to SERPINB6, and
   b/ at least an antibody, or an antigen-binding fragment thereof, specifically binding to EGFR, and
   c/ at least an antibody, or an antigen-binding fragment thereof, specifically binding to CEACAM6, and
   d/ at least an antibody, or an antigen-binding fragment thereof, specifically binding to RNPEP;

with the cancer being selected from the group consisting of myeloma; melanoma; breast cancer, in particular triple negative breast cancer; prostate cancer; primary and metastatic colorectal cancer, in particular colon cancer or metastatic colon cancer; lung cancer, in particular non small cell lung cancer (NSCLC) or small cell lung cancer (SCLC); liver cancer, in particular hepatocarcinoma or cholangiocarcinoma; primary and metastatic pancreas cancer, in particular pancreatic adenocarcinoma; lymphoma, in particular B-cell or T-cell lymphoma, more particularly T-cell lymphoma; gastric cancer; head and neck cancer; ovarian cancer; sarcoma; acute and chronic leukemia; osteosarcoma; anal cancer; testicular cancer; uterus cancer; thyroid cancer; cancer of the central nervous system; gastrointestinal stromal cancer; epidermal carcinoma and oesophageal cancer. In particular, the cancer may be selected from the group consisting of pancreas cancer, in particular pancreatic adenocarcinoma; liver cancer, in particular hepatocarcinoma or cholangiocarcinoma; colorectal cancer, in particular metastatic colon cancer; and lung cancer, in particular non small cell lung cancer (NSCLC). In particular, the cancer is selected from the group consisting of pancreas cancer, in particular pancreatic adenocarcinoma; liver cancer, in particular hepatocellular carcinoma; and colorectal cancer, in particular colon cancer, more particularly metastatic colon cancer.

A cancer as considered in the present invention may in particular comprise cancer cells harboring at least one KRAS mutation. The at least one KRAS mutation may in particular be selected from the group consisting of KRAS^{G12A}, KRAS^{G12C}, KRAS^{G12D}, KRAS^{G12S}, KRAS^{G12V}, KRAS^{G13C} and KRAS^{G13D}; in particular is selected from the group consisting of KRAS^{G12D}, KRAS^{G12C} and KRAS^{G12V}; more particularly is KRAS^{G12D},

In particular, the cancer is pancreas cancer, in particular pancreatic adenocarcinoma.

In a particular embodiment, a combination for use according to the invention is such that it comprises:
(i) at least a first antibody, or an antigen-binding fragment thereof, specifically binding to MVP; and
(ii) a/at least an antibody, or an antigen-binding fragment thereof, specifically binding to BCAP31, and
   b/ at least an antibody, or an antigen-binding fragment thereof, specifically binding to PEPD, and
   c/ at least an antibody, or an antigen-binding fragment thereof, specifically binding to PAXX, and
   d/ at least an antibody, or an antigen-binding fragment thereof, specifically binding to ASPH, and
   e/ at least an antibody, or an antigen-binding fragment thereof, specifically binding to mutated KRAS subtype KRAS^{G12D};
   with the cancer being pancreas cancer, in particular pancreatic adenocarcinoma.

In a particular embodiment, a combination for use according to the invention is such that it comprises:
(i) at least a first antibody, or an antigen-binding fragment thereof, specifically binding to MVP; and
(ii) a/ at least an antibody, or an antigen-binding fragment thereof, specifically binding to SERPINB6, and
   b/ at least an antibody, or an antigen-binding fragment thereof, specifically binding to EGFR, and
   c/ at least an antibody, or an antigen-binding fragment thereof, specifically binding to CEACAM6, and
   d/ at least an antibody, or an antigen-binding fragment thereof, specifically binding to RNPEP;
with the cancer being pancreas cancer, in particular pancreatic adenocarcinoma.

The present text further provides the use of a combination of antibodies, or antigen-binding fragments thereof, in the manufacture of a medicament, the combination of antibodies, or antigen-binding fragments thereof, being as defined above.

The present text further provides a method for treating cancer in an individual in need thereof, comprising at least the step of administering, to said individual, a combination of antibodies, or antigen-binding fragments thereof, comprising:
(i) at least a first antibody, or an antigen-binding fragment thereof, specifically binding to MVP (Major vault protein); and
(ii) at least an antibody (a), or an antigen-binding fragment thereof, specifically binding to at least an antigen selected from the group consisting of BCAP31 (B cell receptor-associated protein 31), PEPD (Peptidase D), PAXX (Paralog of x-ray repair cross-complementing protein 4 (XRCC4) and XRCC4-like factor (XLF)), ASPH (Aspartate β-hydroxylase) and mutated KRAS (Kirsten rat sarcoma viral oncogene homologue) subtype KRAS^{G12D}; and/or, in particular or,
(iii) at least an antibody (b), or an antigen-binding fragment thereof, specifically binding to at least an antigen selected from the group consisting of SERPINB6 (Serine protease inhibitor B6), EGFR (Epidermal growth factor receptor), CEACAM6 (Carcinoembryonic antigen-related cell adhesion molecule 6) and RNPEP (Arginyl aminopeptidase (aminopeptidase B)).

An individual in need thereof is an individual who suffers from cancer. In particular a cancer as described above.

The present text also describes a pharmaceutical composition for treatment of cancers, comprising a combination of antibodies, or antigen-binding fragments thereof, said combination of antibodies, or antigen-binding fragments thereof, comprising:
(i) at least a first antibody, or an antigen-binding fragment thereof, specifically binding to MVP (Major vault protein); and
(ii) at least an antibody (a), or an antigen-binding fragment thereof, specifically binding to at least an antigen selected from the group consisting of BCAP31 (B cell receptor-associated protein 31), PEPD (Peptidase D), PAXX (Paralog of x-ray repair cross-complementing protein 4 (XRCC4) and XRCC4-like factor (XLF)), ASPH (Aspartate β-hydroxylase) and mutated KRAS (Kirsten rat sarcoma viral oncogene homologue) subtype KRAS^{G12D}; and/or, in particular or,
(iii) at least an antibody (b), or an antigen-binding fragment thereof, specifically binding to at least an antigen selected from the group consisting of SERPINB6 (Serine protease inhibitor B6), EGFR (Epidermal growth factor receptor), CEACAM6 (Carcinoembryonic antigen-related cell adhesion molecule 6) and RNPEP ((Arginyl aminopeptidase (aminopeptidase B)).

The combination of antibodies, or antigen-binding fragments thereof and the cancer may be as defined above.

The present disclosure is further illustrated, without in any way being limited to, by the Examples hereafter.

### Examples

### Example 1: In vitro cytotoxicity study of two combinations of antibodies of the invention in different human cancer cell lines (CDC assay)

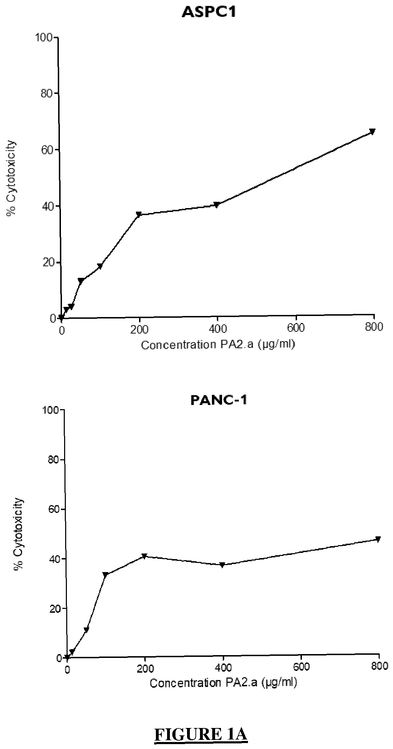

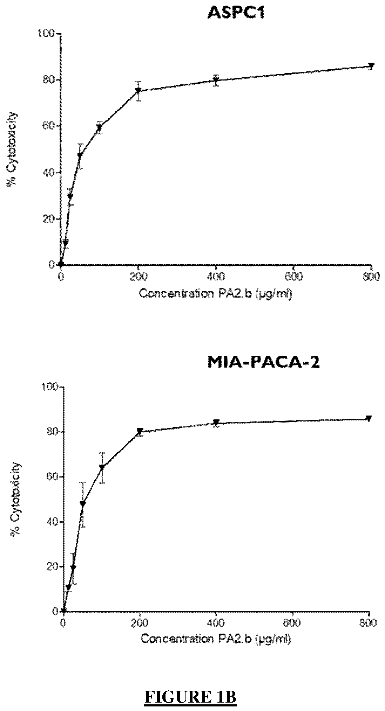

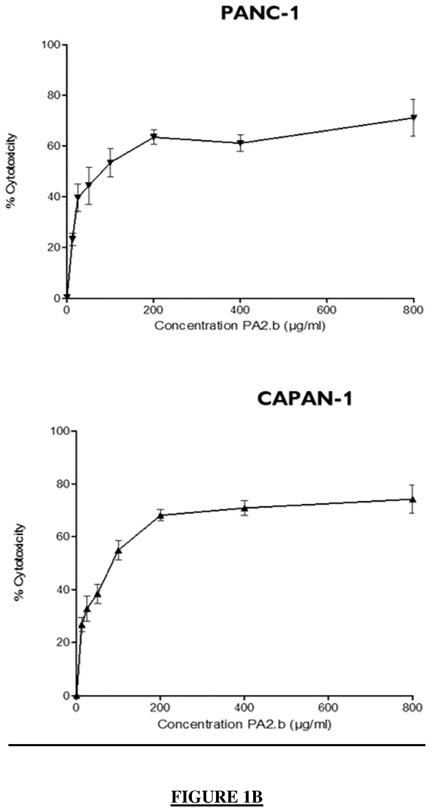

Different pancreatic cancer cell lines were exposed to combinations of antibodies of the invention, specifically the polyclonal antibody compositions "PA2.a" (Figure 1A) or "PA2.b" (Figure 1B), at serial concentrations ranging from 12.5 µg/mL to 800 µg/mL, in the presence of rabbit complement (1:3 final dilution) (n = 3).

The solid cancer cell lines tested were as follows:
- the ASPC1 and PANC-1 cell lines are carrying the mutation KRAS^{G12D}, present in 39.71% of pancreatic adenocarcinoma,
- the CAPAN-1 cell line is carrying the mutation KRAS^{G12V}, present in 31.68% of pancreatic adenocarcinoma, and
- the MIA-PACA-2 cell line is carrying the mutation KRAS^{G12C}, present in 1.74% of pancreatic adenocarcinoma.

After 1 hour of incubation at 37°C, cell viability was assessed by using the NucleoCounter^{®} NC-3000^{™} Advanced Image Cytometer (Chemometec, Denmark).

### Results:

The results show that PA2.a and PA2.b induced a strong cytotoxicity against pancreatic cancer cell lines ranging respectively from 47% to 65% at EC_{Max} 800 µg/mL (Figure 1A) and from 63% to 80% at EC_{Max} 200 µg/mL (Figure 1B).

### Example 2: Global cytotoxicity of two combinations of antibodies of the invention in different human solid tumor cell lines after 24 hours incubation

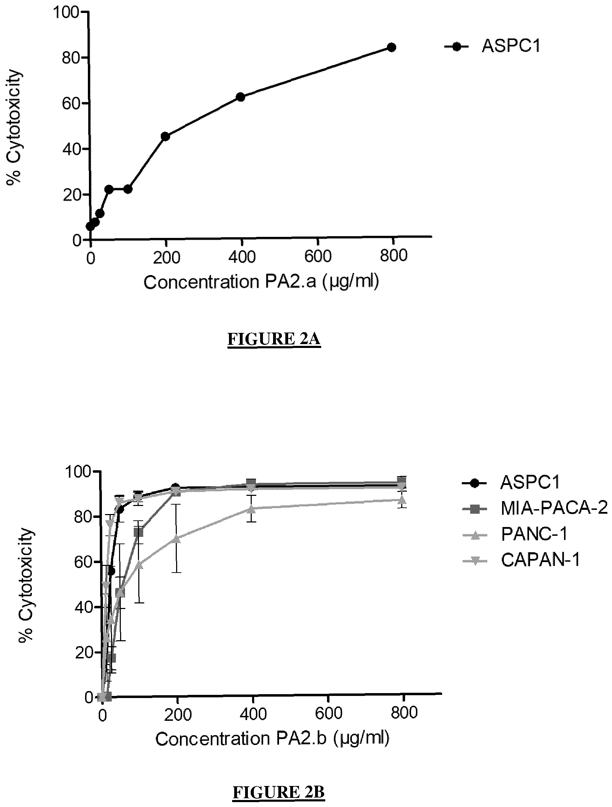

The ASPC1, PANC-1, CAPAN-1 and MIA-PACA-2 pancreatic adenocarcinoma cell lines were exposed to the anti-tumor polyclonal antibody compositions PA2.a (Figure 2A) or PA2.b (Figure 2B) of the invention at serial concentrations ranging from 12.5 µg/mL to 800 µg/mL, and in the presence of rabbit complement (1:3 final dilution) (n = 3).

After 24 hours of incubation at 37°C, cell viability was determined using the CellTiter-Glo@ Cell Viability Assay. Cell viability was quantified by the CellTiter-Glo^{®} One Solution cell viability assay (Promega G8462). After incubation of the cells, the CellTiter-Glo@ One Solution Assay reagent was brought to ambient temperature. Next, 100 µL of CellTiter-Glo@ One Solution Assay reagent were added to each well. Plates were shaken for 2 minutes to induce cell lysis and incubated for 20 minutes prior to reading luminescence (LU) by using the GloMax plate reader (Promega).

### Results:

Global cytotoxicity was induced by the polyclonal antibody compositions PA2.a and PA2.b in a dose-dependent manner across all tested pancreatic cancer cell lines. Notably, cytotoxicity levels in cells incubated with PA2.a or PA2.b ranged from 86 % to 94 % at a concentration of 800 µg/mL (Figures 2A and 2B).

### Example 3: Apoptotic activity of a combination of antibodies of the invention in different human pancreatic cancer cell lines

ASPC-1, MIA-PACA-2 and CAPAN-1 cell lines (300 000 cells per well) were exposed to a concentration of 300 µg/mL of the anti-tumor polyclonal antibody composition PA2.b of the invention.

After 20 hours of incubation at 37°C, Annexin V-CF488A conjugated and Hoechst 33342 (final concentration: 10 µg/mL) were added and incubated for 15 minutes at 37°C. After washes, cell pellets were suspended in 100 µL Annexin V binding buffer supplemented with 10 µg/mL Propidium iodure. Apoptotic cells were analysed using NucleoCounter^{®} NC-3000^{™} Advanced Image Cytometer (Chemometec, Denmark).

### Results:

As demonstrated in Table 1 below, PA2.b induced an apoptotic activity in all the tested pancreatic adenocarcinoma cell lines, with percentages of cell death ranging from 36.76% to 68.01% at a concentration of 300 µg/mL.

**Table 1**

| | | **ASPC-1** | **MIA-PACA-2** | **CAPAN-1** |
|---|---|---|---|---|
| % cell death | PA2.b 300 µg/mL | 56% | 68.01% | 36.76% |

### Example 4: Comparison between the global cytotoxicity of a combination of antibodies of the invention and gemcitabine in different cell lines

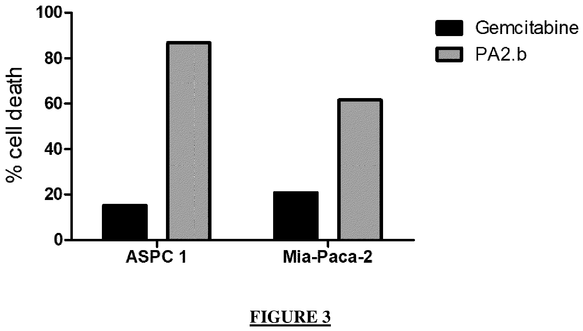

The cell death induced by PA2.b, a polyclonal antibody composition of the invention, was compared with the cell death induced by gemcitabine, a pyrimidine antimetabolite used in the treatment of pancreatic cancer, in two pancreatic adenocarcinoma cell lines (Figure 3).

Cytotoxic activity was evaluated on the ASPC1 and MIA-PACA-2 cell lines. The cells were incubated for 30 minutes at 37°C in the presence of PA2.b or gemcitabine at a concentration of 100 µg/mL with rabbit complement diluted to final 1:3 and 120 µM, respectively (100 000 tumoral cells per well).

After 24 hours of incubation at 37°C, cell viability was determined using the CellTiter-Glo@ Cell Viability Assay. Cell viability was quantified by the CellTiter-Glo^{®} One Solution cell viability assay (Promega G8462). After incubation of the cells, the CellTiter-Glo@ One Solution Assay reagent was brought to ambient temperature. Next, 100 µL of CellTiter-Glo@ One Solution Assay reagent were added to each well. Plates were shaken for 2 minutes to induce cell lysis and incubated for 20 minutes prior to reading luminescence (LU) by using the GloMax plate reader (Promega).

### Results:

It was demonstrated that PA2.b, at a moderate concentration of 100 µg/mL, induced higher cell death (ranging from 63% to 85%) than gemcitabine in the tested adenocarcinoma cell lines (Figure 3).

In contrast, gemcitabine, although used at high concentration (120 µM), induced mild cell death (around 20%) in the two tested cell lines.

### Example 5: Comparison of pancreatic cancer spheroid formation in vitro after treatment with a combination of antibodies of the invention or gemcitabine

ASPC1 cells were treated with serial concentrations of the PA2.b polyclonal antibody composition according to the invention (11, 33, 100 or 300 µg/mL) or gemcitabine (100 µM) for 24 hours in the presence of rabbit complement (1:6 final dilution).

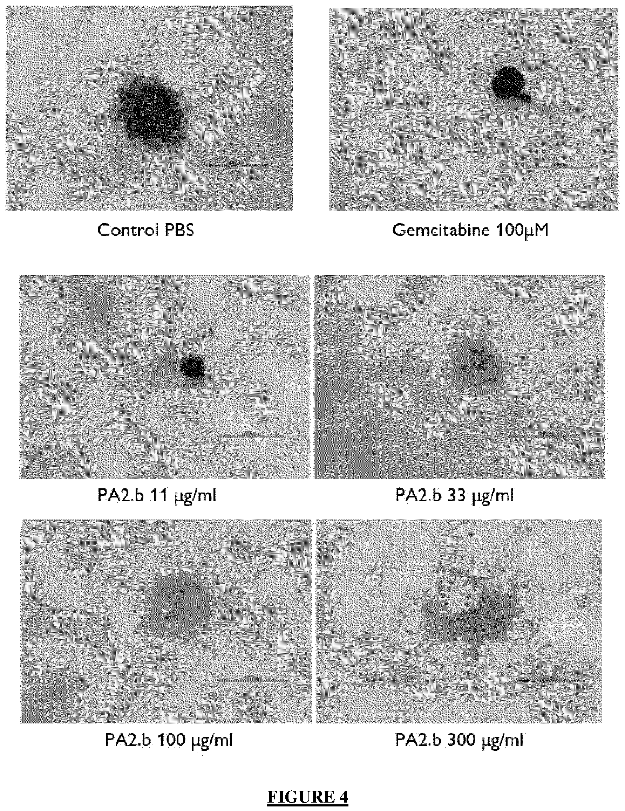

Cell viability was assessed using the NucleoCounter^{®} NC-3000^{™} Advanced Image Cytometer (Chemometec, Denmark). 1000 live treated ASPC1 cells were cultured in 2.5% Matrigel in a Nunclon Sphera 96-well plate, then centrifuged for 10 minutes at 1500 rpm at room temperature and incubated at 37°C. Sphere formation was then followed for all conditions (Figure 4).

### Results:

The results showed that PA2.b reduced the tumorigenicity of ASPC1 cells, unlike gemcitabine, by blocking sphere formation as early as 33 µg/mL (Figure 4).

### Example 6: Anti-tumor activity of a combination of antibodies of the invention in ASPC1 3D culture

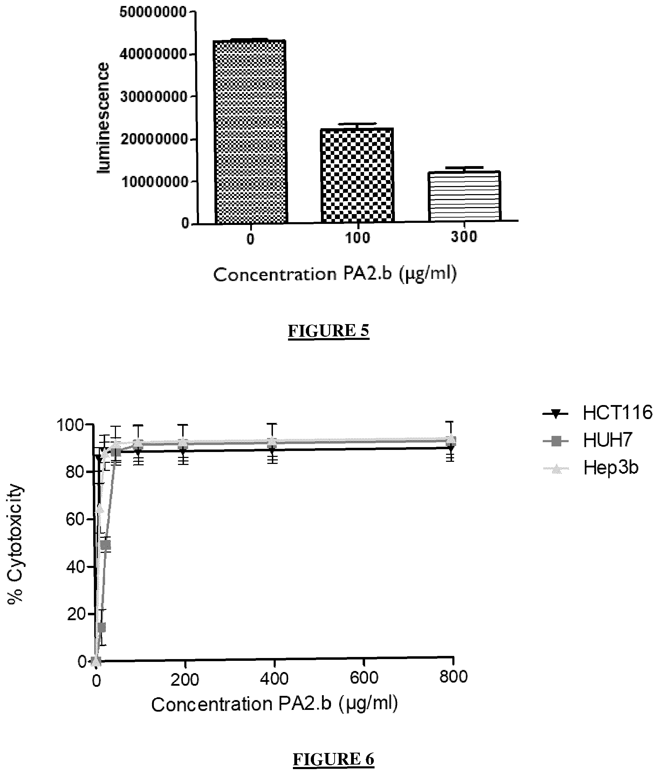

1000 live ASPC1 cells were cultured in 2.5% Matrigel in a Nunclon Sphera 96-well plate, then centrifuged for 10 minutes at 1500 rpm at room temperature and incubated at 37°C (day 0). Sphere formation was then followed for 10 days (Figure 5).

At days 3 and 7 of culture, spheres were treated with the polyclonal antibody composition PA2.b at concentrations of 100 µg/mL or 300 µg/mL, in the presence of rabbit complement (1:6 final dilution). Control spheres were also cultured in complete medium supplemented with rabbit complement (1:6 final dilution).

### Results:

The results demonstrated that PA2.b, after 10 days of culture, reduced pancreatic spheroid tumor growth in a dose-dependent manner. Sphere formation was reduced by 49.11% at a PA2.b concentration of 100 µg/mL and by 73.35% at a concentration of 300 µg/mL (Figure 5).

### Example 7: In vitro global toxicity study of a combination of antibodies of the invention in hepatocellular and colorectal cancers cell lines

Tumor cells representative of hepatocellular carcinoma (HUH7 and Hep3b) and colorectal cancer (HCT116) were exposed to the anti-tumor polyclonal antibody composition PA2.b at serial concentrations ranging from 12.5 µg/mL to 800 µg/mL in the presence of rabbit complement (1:3 final dilution) (Figure 6).

After 24 hours of incubation at 37°C, cell viability was determined using the CellTiter-Glo@ Cell Viability Assay. Cell viability was quantified by the CellTiter-Glo^{®} One Solution cell viability assay (Promega G8462). After cell incubation, the CellTiter-Glo^{®} One Solution Assay reagent was brought to ambient temperature. Next, 100 µL of CellTiter-Glo@ One Solution Assay reagent were added to each well. Plates were shaken for 2 minutes to induce cell lysis and incubated for 20 minutes prior to reading luminescence (LU) by using the GloMax plate reader (Promega).

### Results:

The results demonstrated that the polyclonal antibody composition of the invention, PA2.b, induced strong cytotoxicity in all tested hepatocellular and colorectal cancer cell lines, even at the low concentration of 50 µg/mL (Figure 6).

### Example 8: In vivo anti-tumor activity of a combination of antibodies of the invention in a HUH7 xenograft mouse model

A xenograft *in vivo* mouse model of hepatocellular carcinoma was obtained by the subcutaneous injection of 5,000,000 HUH7 cells at day 0 in 50% Matrigel.

Three groups of mice were included in this study (n = 10 mice per group): a control group without treatment, a treated group with a combination of antibodies of the invention (PA2.a), and a treated group with sorafenib, a multikinase inhibitor used in the

treatment of liver cancers. Treatment was initiated from the beginning of tumor growth and occurred every 2 to 3 days for a total duration of 28 days. Treatment consisted in the intraperitoneal injection of PA2.a at 40 mg/kg, or in the intraperitoneal injection of sorafenib at a dose of 40 mg/kg 5 days out of 7.

### Results:

The results showed that, after 25 days of treatment, the polyclonal antibody composition PA2.a slowed down tumor progression and reduced tumor growth by 52% compared to untreated mice, and by 32% compared to the mice that received sorafenib.

### Example 9: Targets of a combination of antibodies of the invention

HuProt^{™} array (CDI Labs, USA) was used for the assay of two polyclonal antibody compositions of the invention, respectively named PA2.a and PA2.b. After blocking, the array was probed with the polyclonal antibody compositions (1 µg/mL) at room temperature for 1 hour. Then, the array was washed three times with TBST for 10 minutes and probed with Alexa647-anti-swine IgG secondary antibody under conditions optimized by CDI Labs for signal detection.

Z score is the average Z score of the duplicate spots of a given protein (each protein is printed in duplicate on a HuProtTM array). The Z score of each spot on a given array is calculated according to the algorithm below: Z= [F635 - F635(avg)] / F635(std). F635(avg) and F635(std) are the average and standard deviation of the F635 values of all spots on the array, respectively.

### Results:

Binding of the polyclonal antibody compositions of the invention was observed for the following protein targets:

- MVP, BCAP31, PEPD, PAXX and ASPH for PA2.a, and
- MVP, SERPIN B6, EGFR, CEACAM6 and RNPEP for PA2.b.

### Example 10: Binding of the polyclonal antibody composition of the invention PA2.a to KRAS^{G12D}

To assess recognition of PA2.a on KRAS^{G12D}, recombinant KRAS^{G12D} protein was coated onto a MAXISORP 96-well plate at a concentration of 1 µg/mL (50 µL per well) in carbonate/bicarbonate buffer and incubated overnight at 4°C. Saturation was carried out with a solution of PBS/tween 20 0.05%/BSA 2% at 100 µL per well and incubated for 2 hours at room temperature. After 3 washes in PBS/tween 20 0.05%, PA2.a was added at a concentration of 1000 µg/mL and incubated for 1 hour at room temperature. The secondary antibody used was a protA HRP, diluted at 1/1000 in wash buffer, incubated for 1 hour at room temperature. Revelation was achieved by 15 minutes of incubation in TMB. The reaction was then stopped with H₂SO₄ and read with TECAN at a wavelength of 450 nm.

### Results:

The polyclonal antibody composition PA2.a specifically binds to KRAS^{G12D}.

## Claims

1. A combination of antibodies, or antigen-binding fragments thereof, for use in the treatment of a cancer in a human subject in need thereof, the combination comprising:
(i) at least a first antibody, or an antigen-binding fragment thereof, specifically binding to MVP (Major vault protein); and
(ii) at least an antibody (a), or an antigen-binding fragment thereof, specifically binding to at least an antigen selected from the group consisting of BCAP31 (B cell receptor-associated protein 31), PEPD (Peptidase D), PAXX (Paralog of x-ray repair cross-complementing protein 4 (XRCC4) and XRCC4-like factor (XLF)), ASPH (Aspartate β-hydroxylase) and mutated KRAS (Kirsten rat sarcoma viral oncogene homologue) subtype KRAS^{G12D}; and/or
(iii) at least an antibody (b), or an antigen-binding fragment thereof, specifically binding to at least an antigen selected from the group consisting of SERPINB6 (Serine protease inhibitor B6), EGFR (Epidermal growth factor receptor), CEACAM6 (Carcinoembryonic antigen-related cell adhesion molecule 6) and RNPEP ((Arginyl aminopeptidase (aminopeptidase B)).

2. The combination for use according to claim 1, comprising:
(i) at least a first antibody, or an antigen-binding fragment thereof, specifically binding to MVP; and
(ii) at least an antibody (a), or an antigen-binding fragment thereof, specifically binding to at least an antigen selected from the group consisting of BCAP31 (B cell receptor-associated protein 31), PEPD (Peptidase D), PAXX (Paralog of x-ray repair cross-complementing protein 4 (XRCC4) and XRCC4-like factor (XLF)), ASPH (Aspartate β-hydroxylase) and mutated KRAS (Kirsten rat sarcoma viral oncogene homologue) subtype KRAS^{G12D}; or
(iii) at least an antibody (b), or an antigen-binding fragment thereof, specifically binding to at least an antigen selected from the group consisting of SERPINB6 (Serine protease inhibitor B6), EGFR (Epidermal growth factor receptor), CEACAM6 (Carcinoembryonic antigen-related cell adhesion molecule 6) and RNPEP (Arginyl aminopeptidase (aminopeptidase B)).

3. The combination for use according to claim 1 or 2, comprising:
(i) at least a first antibody, or an antigen-binding fragment thereof, specifically binding to MVP; and
(ii) a/ at least an antibody, or an antigen-binding fragment thereof, specifically binding to BCAP31, and
b/ at least an antibody, or an antigen-binding fragment thereof, specifically binding to PEPD, and
c/ at least an antibody, or an antigen-binding fragment thereof, specifically binding to PAXX, and
d/ at least an antibody, or an antigen-binding fragment thereof, specifically binding to ASPH, and
e/ at least an antibody, or an antigen-binding fragment thereof, specifically binding to mutated KRAS subtype KRAS^{G12D}; and/or
(iii) a/ at least an antibody, or an antigen-binding fragment thereof, specifically binding to SERPINB6, and
b/ at least an antibody, or an antigen-binding fragment thereof, specifically binding to EGFR, and
c/ at least an antibody, or an antigen-binding fragment thereof, specifically binding to CEACAM6, and
d/ at least an antibody, or an antigen-binding fragment thereof, specifically binding to RNPEP.

4. The combination for use according to any one of claims 1 to 3, wherein the antigen-binding fragments of the antibodies present in the combination are independently Fv, Fab, F(ab')2, Fab', dsFv, (dsFv)2, scFv, sc(Fv)2, or diabodies.

5. The combination for use according to any one of claims 1 to 4, wherein the cancer is selected from the group consisting of myeloma; melanoma; breast cancer, in particular triple negative breast cancer; prostate cancer; primary and metastatic colorectal cancer, in particular colon cancer or metastatic colon cancer; lung cancer, in particular non small cell lung cancer (NSCLC) or small cell lung cancer (SCLC); liver cancer, in particular hepatocarcinoma or cholangiocarcinoma; primary and metastatic pancreas cancer, in particular pancreatic adenocarcinoma; lymphoma, in particular B-cell or T-cell lymphoma, more particularly T-cell lymphoma; gastric cancer; head and neck cancer; ovarian cancer; sarcoma; acute and chronic leukemia; osteosarcoma; anal cancer; testicular cancer; uterus cancer; thyroid cancer; cancer of the central nervous system; gastrointestinal stromal cancer; epidermal carcinoma and oesophageal cancer.

6. The combination for use according to any of claims 1 to 5, wherein the cancer is selected from the group consisting of pancreas cancer, in particular pancreatic adenocarcinoma; liver cancer, in particular hepatocarcinoma or cholangiocarcinoma; colorectal cancer, in particular metastatic colon cancer; and lung cancer, in particular non small cell lung cancer (NSCLC).

7. The combination for use according to any of claims 1 to 6, wherein the cancer comprises cancer cells harboring at least one KRAS mutation.

8. The combination for use according to claim 7, wherein the at least one KRAS mutation is selected from the group consisting of KRAS^{G12A}, KRAS^{G12C}, KRAS^{G12D}, KRAS^{G12S}, KRAS^{G12V}, KRAS^{G13C} and KRAS^{G13D}; in particular is selected from the group consisting of KRAS^{G12D}, KRAS^{G12C} and KRAS^{G12V}; more particularly is KRAS^{G12D},

9. The combination for use according to any one of claims 1 to 8, wherein at least one of the antibodies of the combination, and in particular all the antibodies of the combination, is/are devoid of at least one antigenic determinant selected from (i) N-glycolylneuraminic acid (Neu5Gc) and (ii) α-1,3-galactose, and in particular is/are devoid of the two antigenic determinants N-glycolylneuraminic acid (Neu5Gc) and α-1,3-galactose.

10. The combination for use according to any one of claims 1 to 9, wherein at least one of the antibodies of the combination, and in particular all the antibodies of the combination, is/are Immunoglobulin G antibodies.

11. The combination for use according to any one of claims 1 to 10, wherein the combination is a combination of monoclonal antibodies or a polyclonal antibody composition.

12. The combination for use according to claim 11, wherein the polyclonal antibody composition is obtainable by immunization of an animal with pancreatic tumor cell antigens comprising KRAS mutated antigens.

13. The combination for use according to claim 12, wherein the animal is a non-human mammal, in particular a non-human mammal selected from the group consisting of rodents, such as mice, rats, guinea pigs and hamsters; lagomorphs, such as rabbits; ferrets; felines, such as cats; canines, such as dogs; goats; sheep; bovines, such as cows; swines, such as pigs and hogs; camelids; horses; and non-human primates.

14. The combination for use according to claim 13, wherein the non-human mammal is selected from the group consisting of lagomorphs, in particular rabbits; and pigs, and in particular is a rabbit or a pig lacking at least one gene selected in a group comprising (i) a gene encoding a functional cytidine-5'-monophosphate N-acetyl neuraminic acid hydrolase (CMAH) and (ii) a gene encoding a functional α-(1,3)-galactosyltransferase, and more particularly lacking both (i) a gene encoding a functional cytidine-5'-monophosphate N-acetyl neuraminic acid hydrolase (CMAH) and (ii) a gene encoding a functional α-(1,3)-galactosyltransferase.

15. The combination for use according to any one of claims 1 to 4 and 9 to 14, wherein the combination is included in a pharmaceutical composition further comprising a pharmaceutically acceptable carrier.

16. The combination for use according to claim 15, wherein the pharmaceutical composition further comprises at least one anticancer drug different from the antibodies of the combination of antibodies as defined in any one of claims 1 to 4 and 9 to 14.

17. The combination for use according to claim 16, wherein the additional anticancer drug is selected from the group consisting of monoclonal antibodies, in particular selected from the group consisting of anti-CD19, anti-CD20, anti-CD30, anti-CD137, anti-CTLA4, anti-TIM-3, anti-B7-H3, anti-CD123, anti-CD134, anti-CD154, anti-LAG-3, anti-CD227, anti-BTNA3, anti-CD39, anti-CD73, anti-CD115, anti-CD47, anti-SIRP alpha, anti-SIRP gamma, anti-CD28, anti-NCR, anti-NKp46, anti-NKp30, anti-NKp44, anti-NKG2D, anti-PD1, anti-PDL1, mogamulizumab, obinutuzumab, polatuzumab vedotin, Yttrium Y 90-ibritumomab tiuxetan, mosunetuzumab, cetuximab, atezolizumab/bevacizumab, anti-VEGF antibodies, anti-DNAM-1 monoclonal antibodies and mixtures thereof.

18. The combination for use according to any one of claims 15 to 17, wherein the pharmaceutical composition further comprises at least one chemotherapy treatment, in particular a chemotherapy treatment selected from the group consisting of a chemotherapy regimen consisting of Cyclophosphamide, Hydroxydaunorubicin, Oncovin and Prednisone (CHOP); a chemotherapy regimen consisting of Cyclophosphamide, Hydroxydaunorubicin, Oncovin, Etoposide and Prednisone (CHOEP); HDAC inhibitors; ibrutinib; acalabrutinib; zanubrutinib; copanlisib; venetoclax; folfirinox; cisplatin; doxorubicin; irinotecan; liposomal irinotecan; oxaliplatin; Nap paclitaxel; paclitaxel; gemcitabine; sorafenib; lenvatinib; imatinib; sunitinib; regorafenib; mTOR inhibitors and mixtures thereof.

19. The combination for use according to any one of claims 1 to 18, for use in providing at least an antitumoral activity selected from the group consisting of complement dependent cytotoxicity (CDC), global cytotoxicity, apoptotic activity, impaired spheroid formation, decreased tumor size, tumor invasion and/or metastasis.
